## Europäisches Patentamt
## European Patent Office
## Office européen des brevets

(11) Numéro de publication: **0 124 388**
**A1**

# DEMANDE DE BREVET EUROPEEN

(21) Numéro de dépôt: 84400545.4

(22) Date de dépôt: 16.03.84

(51) Int. Cl.³: **C 12 N 1/14**, C 12 P 21/02, C 12 P 17/06, C 07 C 103/52 // (C12N1/14, C12R1/885),(C12P21/02, C12R1/885),(C12P17/06, C12R1/885)

(30) Priorité: 28.04.83 FR 8307051

(43) Date de publication de la demande: 07.11.84 Bulletin 84/45

(84) Etats contractants désignés: **AT BE CH DE FR GB IT LI LU NL SE**

(71) Demandeur: **LES PRODUITS ORGANIQUES DU SANTERRE ORSAN, 16 rue Ballu, F-75009 Paris (FR)**

(72) Inventeur: **Merlier, Odile Anne-Marie, 5 rue de Nesle Mesnil St.Nicaise, F-80190 Nesle (FR)**
Inventeur: **Boirie, Monique Jeanne, 37 rue de l'Avenir Levis Saint Nom, F-78320 Le Mesnil Saint Denis (FR)**
Inventeur: **Pons, Benoit Joseph, Rue des Marronniers Languevoisin, F-80190 Nesle (FR)**
Inventeur: **Renaud, Claude Marcel, 6 Fg Saint Léonard, F-80190 Nesle (FR)**

(74) Mandataire: **Portal, Gérard et al, Cabinet Z. Weinstein 20, Avenue de Friedland, F-75008 Paris (FR)**

(54) Nouvelle souche de Trichoderma harzianum procédé d'isolement de cette souche, procédé de culture de cette souche, nouveaux peptides produits par cette souche, et application de cette souche et ces nouveaux peptides ou du produit obtenu par le procédé de culture comme moyen de lutte biologique sous forme de produit phytosanitaire.

(57) L'invention concerne une nouvelle souche de Trichoderma harzianum.

Cette nouvelle souche est déposée à l'Institut Pasteur sous le N° I 223 et à l'ATCC sous le N° 20672. Cette souche a été isolée par une technique de clonage à partir de champignons de Trichoderma harzianum à partir d'un sol humide comme un sol maraicher ou un sol cultivé.

Cette nouvelle souche ainsi que les peptides qu'elle produit dénommés Trichorzianines sont utilisés comme moyen de lutte biologique en particulier pour le traitement de la pourriture grise de la vigne ou la maladie du plomb des arbres fruitiers.

"Nouvelle souche de Trichoderma harzianum,procédé d'isolement de cette souche, procédé de culture de cette souche, nouveaux peptides ou composés produits par cette souche, et application de cette souche et ces nouveaux peptides ou du produit obtenu par le procédé de culture comme moyen de lutte biologique sous forme de produit phytosanitaire"

La présente invention concerne essentiellement une nouvelle souche de Trichoderma harzianum, un procédé d'isolement de cette souche, un procédé de culture de cette souche, de nouveaux peptides ou composés produits par cette souche nouvellement dénommés Trichorzianines et l'application de cette souche, des nouveaux peptides ou du produit obtenu par le procédé de culture comme moyen de lutte biologique, en particulier sous forme de produit phytosanitaire. Plus précisément, cette nouvelle souche de Trichoderma harzianum peut être utilisée comme moyen de lutte biologique, en particulier sous forme de produit phytosanitaire, contre le champignon parasite Botrytis cinerea (pourriture grise de la vigne) ou contre le champignon parasite Stereum purpureum (maladie du plomb des arbres fruitiers) ou encore contre Ceratocystis ulmi (graphiose de l'orme) ou encore contre le champignon parasite Sclerotinia (sclérotiniose) ou encore contre le champignon parasite Stereum hirsutum, exemples non limitatifs.

La nouvelle souche de Trichoderma harzianum isolée par la demanderesse a fait l'objet d'un dépôt auprès de l'Institut Pasteur le 2 Mars 1983 sous le N°I 223 ainsi que d'un dépôt auprès de l'American Type Culture Collection, en abrégé ATCC sous le N° ATCC 20672 le 22 Avril 1983.

Cette nouvelle souche I223 a été reconnue comme étant

2

un champignon Trichoderma harzianum selon la classification Rifai.

Cette nouvelle souche de Trichoderma harzianum I223 présente en effet l'aspect cultural suivant:

-un mycélium ras, translucide à peine discernable à l'oeil nu, à la surface duquel apparaissent au bout de trois jours environ de culture, des formations sporogènes d'abord blanches qui se colorent par la suite en vert sombre. La structure sporifère est la suivante : les conidiophores portent des branches ramifiées de façon orthogonale, les cellules sporogènes (phialides) portées par ces dernières peuvent être groupées par deux ou trois, elles sont de taille variable (8 à 12 microns), étroites (2,5 microns), peu ventrues et souvent recourbées, les spores qu'elles portent sont globuleuses à sub-globuleuses, colorées en vert et mesurent en moyenne 3 microns de diamètre, elles apparaissent lisses au grandissement 1000. La paroi des spores est complètement lisse au microscope électronique.

La température de croissance est de 17 à 32°C avec un optimum de 20 à 24°C. Elles poussent sur milieu classique, c'est-à-dire malt-agar; pomme de terre-glucose-agar; flocons d'avoine; et à un pH compris entre 4 et 7 avec un optimum vers 5.

La sporulation est plus importante sur un milieu riche et sa croissance est aérobie.

Le procédé d'isolement de cette nouvelle souche de Trichoderma harzianum I223 ou ATCC N° 20672 a pour but d'obtenir une souche :

-présentant une croissance rapide, la souche doit se développer rapidement afin d'être efficace vis à vis des parasites,

-ayant une bonne croissance en milieu pauvre,

-se développant à température assez basse (15-17°C),

-ayant la plus importante production de spores par unité de milieu gélosé,

-ayant la plus importante production de substance antifongique par unité de poids de spores, production que l'on peut évaluer

.soit par comparaison des pouvoirs antagonistes à distance (comme il sera expliqué plus loin à l'exemple 7)

.soit par extraction aux solvants de ces substances. Les meilleures souches obtenues permettent d'obtenir une quantité de substances de 2% (exprimée en poids de substance par poids de produit final)

-ayant le meilleur pouvoir antagoniste par contact.

Ce procédé d'isolement est caractérisé en ce qu'il comprend les étapes successives suivantes :

A - on prélève des champignons Trichoderma harzianum dans un endroit approprié, c'est-à-dire connu pour pouvoir contenir des champignons Trichoderma harzianum, tel que sol humide comme un sol maraîcher ou un sol cultivé;

B - on fait croître les champignons de Trichoderma harzianum prélevés, sur un milieu de culture gélosé en boîte de Pétri à 24°C à la lumière pendant 3 à 7 jours;

C - on prélève le maximum de spores de Trichoderma harzianum de couleur verte;

D - on met en suspension dans l'eau ce prélèvement de Trichoderma harzianum pour dissocier les spores;

E - on détermine la ou les meilleures souches parmi les souches dissociées par un programme de sélection par clonage de préférence réalisé comme suit :

a) on effectue une culture séparée de chaque spore dissociée sur un milieu gélosé riche tel que milieu aux flocons d'avoine en boîte de Pétri;

b) au bout de 48 heures, on remet en suspension la totalité des spores de chaque boîte prise séparément, et on ajuste le titre à $10^6$ spores/millilitre;

c) on prélève une ou plusieurs gouttes de suspension de souche obtenue après l'étape b) que l'on inocule dans l'un des milieux suivants :

i. un milieu gélosé pauvre, par exemple à l'extrait de malt à 20 grammes par litre en boîte de Pétri et que l'on cultive à 24°C;

ii. un milieu riche, par exemple aux flocons d'avoine à 30 grammes par litre, que l'on cultive à une température assez basse, par exemple de 15 à 17°C;

iii. un milieu de croissance du parasite Botrytis cinerea, par exemple essentiellement à base de concentré de jus de tomate, préalablement ensemencé du parasite lui-même Botrytis cinerea, en vue de réaliser un essai de pouvoir antagoniste par contact, et à distance, avec culture à 24°C;

iiii. un milieu riche, par exemple aux flocons d'avoine à 30 grammes par litre que l'on cultive à 24°C, en vue de déterminer les souches ayant la production la plus importante de spores par unité de surface de milieu gélosé,

d) on observe la croissance et le comportement des souches à partir de 48 heures, toutes les 24 heures, et on évalue selon des critères connus en soi la qualité de cette croissance;

e) on retient la ou les souches ayant obtenu la meilleure performance sur l'ensemble des essais de l'étape c) ci-dessus et produisant la plus grande quantité de peptides; cette souche constitue la souche déposée auprès de l'Institut Pasteur comme nouvelle souche Trichoderma harzianum I223, également déposée auprès de l'ATCC sous le N° ATCC 20672.

La présente invention concerne également un procédé de culture de la souche ou des souches de Trichoderma harzianum ainsi sélectionnées, et de préférence de la souche Trichoderma harzianum I223 ou ATCC 20672, caractérisé en ce qu'il consiste à cultiver la ou lesdites souches de Trichoderma harzianum, et de préférence Trichoderma harzianum I223 ou ATCC N° 20672 sur un milieu liquide, composé de constituants nutritifs habituels en fermentation, stérilement, en couches fines sur un plateau et à la lumière pendant un temps de fermentation tel que les spores atteignent leur maturité; à la fin de la fermentation, on sèche le produit total que l'on broie finement et que l'on tamise de manière à obtenir une poudre sèche et fine ayant de préférence un diamètre inférieur à 200 microns, contenant les spores viables, et pouvant se mettre en suspension dans l'eau et être pulvérisée.

Ce produit de base titre de préférence au moins $10^{10}$ éléments ou spores revivifiables par gramme et peut servir de base à des formulations diverses.

On donne ci-après plusieurs exemples de cultures de la souche Trichoderma harzianum en référence à la souche déposée auprès de l'Institut Pasteur sous le N° I223 ou à l'ATCC sous le N° ATCC 20672

## EXEMPLE 1

On prépare le milieu de culture ou de préculture ayant la composition suivante :

| | |
|---|---|
| glucose | 40 grammes |
| extrait de levure | 5 grammes |
| bactopeptone | 5 grammes |
| $NaNO_3$ | 3 grammes |
| $KH_2PO_4$ | 1 gramme |
| $MgSO_4, 7H_2O$ | 0,5 gramme |
| KCl | 0,5 gramme |
| Eau déminéralisée qsp | 1 litre. |

Le pH naturel de ce milieu est de 5,85 et celui-ci est ajusté à 4,8 par addition de HCl.

On stérilise ce milieu 20 minutes à 121°C puis on ensemence à partir de spores de Trichoderma harzianum I223 ou ATCC N° 20672 obtenues sur milieu gélosé ou à partir d'une préculture réalisée dans le milieu agité ci-dessus décrit.

7

On laisse incuber à 24°C en couches minces, sous lumière permanente ou par intermittence jusqu'à ce que les spores atteignent leur maturité.

On sèche la totalité du milieu et l'on réalise le broyage pour obtenir une poudre sèche et fine ayant de préférence une granulométrie inférieure à 200 microns et qui contient les spores viables de Trichoderma harzianum I223 ou ATCC N° 20672.

EXEMPLE 2

On prépare un milieu de culture ayant la composition suivante :

| | |
|---|---|
| glucose | 5 grammes |
| $KH_2PO_4$ | 0,8 gramme |
| $Ca(PO_4H_2)_2$ | 0,2 gramme |
| $MgSO_4$, $7H_2O$ | 0,5 gramme |
| $MnSO_4$,$5H_2O$ | 0,01 gramme |
| $ZnSO_4$,$7H_2O$ | 0,01 gramme |
| $CuSO_4$,$5H_2O$ | 0,005 gramme |
| $FeSO_4$,$7H_2O$ | 0,001 gramme |
| $KNO_3$ | 0,72 gramme |
| $(NH_4)_2SO4$ | 2 grammes |
| Eau déminéralisée qsp | 1.000 millilitres. |

On effectue une stérilisation de ce milieu pendant 25 minutes à 105°C et ce milieu présente alors un pH égal à 6.

Ce milieu peut être modifié en ajoutant un certain taux de farine de céréale pour obtenir un milieu plus pâteux (par exemple farine de soja, de maïs, d'orge, etc).

Le milieu est ensemencé à partir de spores de Trichoderma harzianum I223 ou ATCC N° 20672 obtenues sur milieu gélosé ou à partir de préculture (de préférence le milieu de l'exemple 1) en milieu liquide et agité.

Après incubation à 24°C en couches minces jusqu'à maturité des spores, on sèche la totalité du milieu et l'on réalise le broyage pour obtenir une poudre fine ayant de préférence une granulométrie inférieure à 200 microns contenant les spores viables, qui est facilement mise en suspension et pulvérisable.

EXEMPLE 3

On prépare le milieu de culture suivant :

| | |
|---|---|
| Flocons d'avoine | 30 grammes |
| Gélose | 20 grammes |
| Eau déminéralisée qsp | 1.000 millilitres. |

Le pH ajusté avec HCl avant la stérilisation de 20 minutes à 121°C doit être après stérilisation de 5,5-5,7.

Lorsque le milieu est chaud, on le répartit en couches minces dans des plateaux.

Après refroidissement, on ensemence en surface à partir d'une préculture (de préférence réalisée avec le milieu de l'exemple 1) qui a été réalisée pendant 18 heures et on laisse incuber à 24°C avec lumière par intermittence ou continuellement.

Après incubation jusqu'à maturité des spores, on racle la surface de la gélose pour récolter les spores formées pour réaliser un séchage et un broyage

9

comme dans les exemples 1 ou 2 précédents. Cette poudre ne contient que des spores viables de Trichoderma harzianum I223 ou ATCC N° 20672 à raison de $8.10^{10}$ spores viables/gramme de poudre.

EXEMPLE 4

On prépare un milieu de culture ayant la composition suivante :

| | |
|---|---|
| Amidon de maïs hydrolysé | 100 grammes |
| Corn steep liquide ... | 40 grammes |
| Acide lactique | 1 gramme |
| Citrate de sodium | 0,5 gramme |
| $KH_2PO_4$ | 0,25 gramme |
| $NaNO_3$ | 0,25 gramme |
| Thiamine | 100 gamma |
| Eau déminéralisée qsp | 1.000 millilitres. |

Le pH après stérilisation de 30 minutes à 121°C doit être de 4-4,2.

Le milieu est préparé dans un fermenteur de 15 litres, type New Brunswick.

L'amidon est hydrolysé partiellement par de l'alpha-amylase pour éviter sa prise en masse après stérilisation et refroidissement.

Après stérilisation, le milieu est mis sous agitation et refroidi. Lorsque la température atteint 24°C, le milieu est ensemencé à raison de 6% en poids avec une préculture de 18 heures d'âge, qui est de préférence réalisée à partir du milieu de l'exemple 1, puis répartie dans de larges plateaux, sur un demi-centimètre d'épaisseur. Ces plateaux sont glissés dans des gaines plastiques scellées à la base et bouchées à l'aide d'un coton à l'autre extrémité. L'incubation est faite à 24°C sous lumière totale ou par intermittence, pendant 7 jours. On sèche la totalité du milieu incubé et on réalise le broyage du produit sec obtenu, de préférence jusqu'à une granulométrie inférieure à 200 microns. Le produit obtenu contient $2.10^{10}$ spores viables par gramme de poudre.

Ainsi, les poudres obtenues dans les différents exemples précédents contiennent $1.10^8$ à $8.10^{10}$ spores par gramme de poudre et dans le cas de l'exemple 3 ou 4, $2.10^{10}$ à $8.10^{10}$ spores par gramme de poudre . Ces spores sont viables et donc en mesure de se reproduire et de multiplier leur activité.

Cette poudre est verte, après broyage et tamisage de granulométrie inférieure à 200 microns. Cette poudre se met facilement en suspension dans l'eau, l'eau utilisée pouvant être de l'eau déminéralisée, de l'eau de pluie, de l'eau de robinet, sans aucun problème de germination des spores.

La suspension ainsi préparée peut être conservée 2 à 3 heures sans problème.

Les essais de décantation effectués montrent qu'en 15 minutes, le taux de décantation est de 0 à 0,5%.

La demanderesse a pu constater d'autre part que ce produit obtenu est incapable d'attaquer la cellulose constitutive de la paroi des végétaux ce qui démontre que le Trichoderma harzianum nouvellement isolé par la demanderesse est non cellulolytique.

En effet, les organismes cellulolytiques sont pourvus

11

de deux enzymes Cl et CX. L'enzyme Cl a une action cellulolytique sur les plantes et cette activité est démontrée par un essai fait sur la cellulose microcristalline Avicel (marque déposée).

Le Trichoderma harzianum I223 ou ATCC N° 20672 ne produit pas ou très peu cette enzyme Cl car il n'y a pas eu d'éclaircissement du milieu soumis à essai de cellulose microcristalline Avicel.

Un autre essai a été réalisé en utilisant de la cellulose régénérée, c'est-à-dire partiellement cristalline, caractérisant la présence des enzymes Cl et CX. Il y a une activité de Trichoderma harzianum I223 ou ATCC N° 20672 sur cette cellulose, ce qui permet de conclure à la présence de l'enzyme CX.

D'après ces deux essais, il apparaît donc que la nouvelle souche de Trichoderma harzianum I223 ou ATCC N° 20672 n'a que l'activité CX et ne produira donc pas de dégâts sur les végétaux sur lesquels elle devra être appliquée pour agir contre les parasites selon les applications qui seront explicitées plus loin.

D'autre part, la présente invention concerne également divers procédés d'extraction des spores produites par Trichoderma harzianum I223 ou ATCC N° 20 672 pour en isoler les peptides ou d'autres substances actives. Le procédé d'isolement des peptides comprend les étapes successives suivantes:

a) introduire les spores de culture de Trichoderma harzianum I223 ou ATCC N° 20 672 de préférence obtenues à partir de l'un quelconque des exemples 1 à 4 précédents, dans de l'acétone pour y dissoudre la partie soluble des spores et obtenir après évaporation de l'acétone un extrait brut.

(Cette première extraction de spores est plus complète à l'éthanol mais les extraits obtenus sont plus complexes que par extraction à l'acétone et donc l'acétone a été de préférence retenue).

b) Soumettre cet extrait brut à au moins une étape d'extraction par l'éther pour en recueillir la fraction insoluble. La fraction soluble est également recueillie car il a été déterminé qu'elle est active contre les champignons parasites tels que Botrytis cinerea . La Demanderesse a isolé la substance principale responsable de cette activité, qui a été identifiée par les examens de ses spectres RMN et de masse comme s'agissant de la pentyl-6 pyrone-2. Cette substance est nouvelle et fait partie intégrante de l'invention. Divers procédés spécifiques d'extraction de la pentyl-6 pyrone-2 seront décrits plus loin en référence à l'exemple 11.

c) Extraire cette fraction insoluble dans l'éther au méthanol pour en recueillir la fraction soluble. La fraction insoluble contient du mannitol ne présentant pas d'activité pour l'application de lutte biologique.

d) Chromatographier la fraction soluble dans le méthanol pour en recueillir la ou les fractions de tête ou fractions lourdes qui contiennent essentiellement les peptides recherchés qui sont nouveaux et qui sont dénommés par la Demanderesse du nom nouveau de Trichorzianines. Ces substances sont actives pour l'application de lutte biologique envisagée comme cela sera démontré plus loin.

La dernière étape d'extraction par chromatographie pour séparer les peptides peut être réalisée sur colonne Séphadex LH20 qui par élution par du
méthanol    permet d'obtenir en tête de colonne
une fraction qui par spectrométrie dans l'infrarouge
présente une absorption à 1 650 cm$^{-1}$ qui est la fréquence de vibration de la liaison peptidique et qui
démontre la présence de peptides.

Pour suivre durant l'extraction et la purification
l'activité des différentes fractions, la Demanderesse
a utilisé le test suivant :

- dans 10 millimètres de milieu gélosé à 45°C tel
que PDA (pomme de terre-dextrose-agar) ou malt agar,
on ajoute l'extrait à tester.

Après agitation, ce milieu est versé dans une boîte
de Pétri de 5 cm de diamètre. Après refroidissement, une
pastille prélevée dans une culture de Botrytis cinerea
en boîte de Pétri est déposée au centre.

Des boîtes témoins sont faites en parallèle dans
lesquelles la même quantité de solvant pur a été introduite pour être certain que la croissance de Botrytis
cinerea peut se faire sans inconvénient.

(Le méthanol est toxique pour la croissance de Botrytis cinerea, l'utilisation de l'éthanol est possible
en faibles quantités ; par contre l'acétone n'a aucun
effet sur la croissance).

L'évolution des cultures est mesurée journellement.

Un exemple d'extraction des peptides ou Trichorzianines à partir des spores de Trichorderma harzianum I223
ou ATCC n° 20672 est donné ci-après.

14

EXEMPLE 5

Exemple d'extraction des Trichorzianines à partir des spores de Trichoderma harzianum I 223 ou ATCC n° 20672

On extrait 1 Kg de spores obtenues à partir de l'exemple 3, par 4 litres d'acétone pendant 24 heures deux fois consécutivement. On obtient une huile jaune dorée après concentration des extraits acétoniques. Dans certains cas, une extraction complémentaire à l'éthanol a été réalisée ( deux fois au moins 48 heures) ; l'extrait obtenu est alors traité de la même façon que l'extrait acétonique.

On reprend cette huile dans l'éther, ce qui donne une fraction polaire insoluble dans l'éther et une fraction apolaire soluble dans l'éther.

La fraction apolaire (55 grammes) soluble dans l'éther, est constituée principalement d'acides gras, de stérols et de pentyl-6 pyrone 2. Cette pentyl-6 pyrone 2 est responsable de l'odeur fruitée des spores et la Demanderesse a pu vérifier qu'elle présente une activité vis-à-vis des champignons parasites et en particulier vis-à-vis de Botrytis cinerea. L'extraction de la pentyl-6 pyrone-2 et la détection de son activité feront l'objet des exemples 11 et 12.

La fraction polaire (14 grammes) insoluble dans l'éther présente une activité puissante contre Botrytis cinerea. Cette fraction insoluble dans l'éther est reprise dans le méthanol, ce qui permet d'éliminer dans la fraction insoluble des sucres, en particulier le mannitol, polyol fréquent dans les champignons.

0124388

15

La fraction insoluble dans le méthanol est soumise à un fractionnement selon la masse moléculaire en la passant sur colonne Sephadex LH20 avec comme éluant le méthanol conformément à la dernière étape d'extraction par chromatographie précitée. Le passage sur Sephadex LH20 constitue une filtration sur un gel de dextrane réticulé.

Les premières molécules éluées sont les plus grosses et sont actives et il s'agit naturellement des nouvelles Trichorzianines formant partie intégrante de l'invention.

Etude de la fraction peptidique

1°) Si on examine cette fraction en chromatographie en couche mince (gel de silice, solvant n-butanol/acétone/eau, 60/20/20 en volume), on obtient trois taches dénommées TCZ 10, TCZ 11, TCZ 13.

La tache TCZ 11, peut être révélée par observation de la plaque en lumière ultraviolette (250 nm). La pulvérisation d'acide sulfurique concentré sur le chromatogramme révèle à froid TCZ 11 qui apparaît en jaune citron et émet une fluorescence jaune sous une lumière UV (366 nm). Le chauffage de la plaque provoque un changement de couleur de la tache qui vire au violet, tandis qu'apparaît une coloration brune pour TCZ 10.

2°) Si l'on réalise une chromatographie préparative des peptides bruts sur colonne de gel de silice (éluant $CH_2Cl_2$, $CH_3OH$, $H_2O$ (65/24/4 en volume), la fraction TCZ 13 très minoritaire est éluée en tête suivie de TCZ 11 et TCZ 10.

La fraction la plus active vis-à-vis de Botrytis cinerea est TCZ 11, la faible activité de TCZ 10 semblant

16

être due à une contamination par TCZ 11.

TCZ 10 a une masse moléculaire assez élevée, c'est un produit polaire, mais une hydrolyse acide qui conduit à sa dégradation ne fait pas apparaître d'acides aminés. Une analyse plus poussée a montré que TCZ 10 était une lécithine.

La Demanderesse s'est intéressée plus à fond à la fraction TCZ 11 majoritaire et très active.

Afin de déterminer s'il s'agissait d'un peptide unique ou d'un mélange, TCZ 11 a été analysé par chromatographie sur plaque de gel de silice greffée (plaque Whatman 200 KC18, éluant en méthanol, eau 95/5 en volume). On obtient deux taches d'intensité différente, mais de même aspect avec les réactifs de révélation :

TCZ 11 A, comme constituant majoritaire avec une valeur Rf égale à 0,60

TCZ 11B avec comme Rf : 0,73.

Les révélations des plaques peuvent faites de plusieurs façons :

- soit par observation de la plaque en lumière ultraviolette : légère absorption qui donne une tache plus sombre,

- soit par pulvérisation de ninhydrine dans l'éthanol puis pulvérisation ensuite d'acide sulfurique concentré.

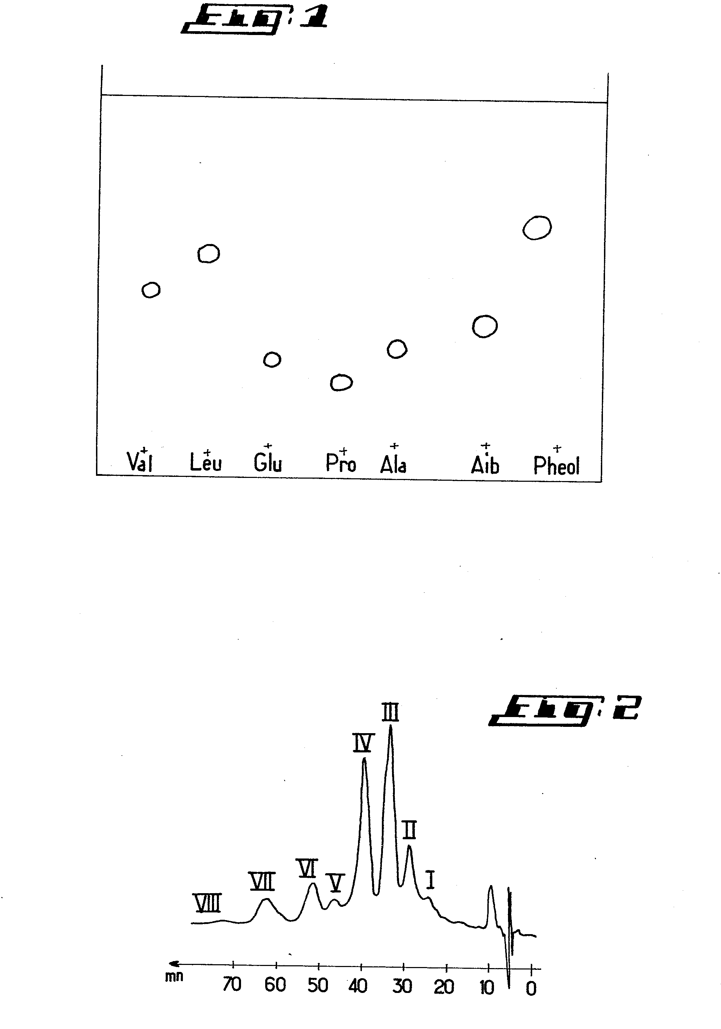

Les TCZ 11 donnent à froid une coloration jaune stable à la lumière. La fraction TCZ 11 complète a été soumise à une hydrolyse acide (HC16N, 24 heures à 110°C) qui la décompose en une série d'amino-acides dont la plupart ont été déterminés par chromatographie sur plaque en couche mince. Le résultat de cette chromatographie fait l'objet de la figure n° 1 annexée. L'éluant est constitué par le mélange butanol/acide acétique/eau (60/20/20 volume/volume). On peut constater à partir des résultats de cette chromatographie que l'hydrolyse acide montre la présence de valine, leucine, acide glutamique, Proline, Alanine, acide alpha-aminoisobutyrique (AIB), Phénylalaninol (PHEOL).

Il est à noter que les produits isolés selon la présente invention sur chromatographie en couche mince de gel de silice avec comme éluant le système BAE précité avec comme rapport volumique 60/20/20 se différencient d'un peptide isolé par la firme américaine UPJHOHN :l'alaméthicine qui ne forme aucune coloration avec le réactif de révélation choisi et déjà cité (ninhydrine puis acide sulfurique concentré).

Cette différence a été vérifiée dans d'autres systèmes de chromatographie.

En résumé, si on est parti de 43 grammes de peptides bruts (obtenus à partir de 2000 grammes de spores) les différentes fractions obtenues par cette chromatographie séparative sont les suivantes :

| Fractions | Poids obtenus (g) |
|-----------|-------------------|
| TCZ 13 | 0,89 |
| TCZ 13 + TCZ 11A | 2,61 |
| TCZ 11A | 5,06 |
| TCZ 11A + Lécithine | 15,66 |
| TCZ 11A + TCZ 11B+Lécithine | 3,90 |
| TCZ 11B + Lécithine | 13,50 |

Tableau 1 - Poids des fractions obtenues au cours de la chromatographie préparative sur colonne de 43 g de peptides bruts (mB 197) ($SiO_2$, CCH).

La fraction TCZ 11A peut être séparé de TCZ 11B par chromatographie sur colonne d'alumine (éluant éthanol à 95 %).

TCZ 11B et la lécithine peuvent être séparés sur colonne de silice (éluant acétone avec des proportions croissantes de méthanol 80/20 jusqu'à 50/50).

De cette façon, la fraction peptidique brute a pu être décomposée en quatre fractions : TCZ 13, TCZ 11A, TCZ 11B, TCZ 10 (lécithine) dont les poids sont donnés dans le tableau suivant :

19

Tableau II

| | Poids estimés (g) |
|---|---|
| TCZ 13 | 2,5 |
| TCZ 11A | 20 |
| TCZ 11B | 10 |
| TCZ 10 | 10 |

Ainsi, le premier fractionnement des peptides bruts est récapitulé schématiquement à la partie I du tableau donné en fin de description page 49 .

Etant donné que la fraction TCZ 11A est la fraction majoritaire et la fraction de loin la plus active, celle-ci a été étudiée d'une façon plus approfondie.

3°)Analyse de la structure de la fraction TCZ 11A

La fraction TCZ 11A a pu être obtenue à l'état cristallin en utilisant comme solvant de cristallisation un mélange acétonitrile et eau (80/20 en rapport volumique). Son point de fusion est de 251-252°C.

La nature peptidique de ce composé a été démontrée par l'analyse de son spectre d'absorption dans l'infrarouge et par son hydrolyse en milieu acide donnant des acides aminés.

Dans le but de vérifier son unicité, des essais de chromatographie haute performance ou haute pression (HPLC) ont été réalisés et ont montré que la fraction TCZ 11A était en fait composée de plusieurs peptides.

Ainsi, après de multiples essais, les conditions convenables d'HPLC ont pu être définies, en choisissant pour phase stationnaire une silice très fine greffée par des hydrocarbures en C 18 (microbondapak C18 dénomination commmerciale) et en utilisant comme éluant, un mélange éthanol-eau (65/35) ou méthanol-eau (78/22)

L'appareil utilisé est un chromatographe Waters, et les peptides élués ont été détectés à 225 nm à l'aide d'un détecteur UV variable Waters M450.

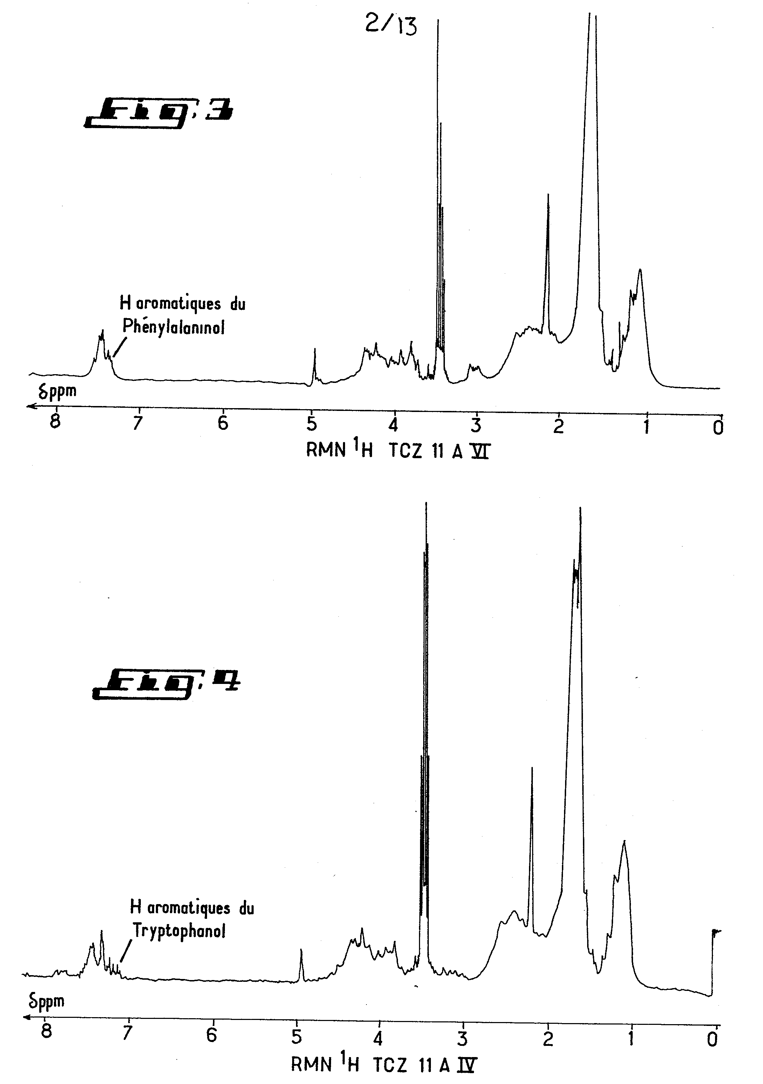

Le chromatogramme obtenu avec la fraction TCZ 11A fait l'objet de la figure 2 et met en évidence les différents peptides composant la fraction TCZ 11A. Ces peptides sont numérotés de I à VII selon l'ordre de sortie de la colonne. Le débit de la colonne HPLC est de 2ml/mn (colonne microbondapak C18 ; 0,39 x 30 cm, granulométrie 9 microns, concentration de l'échantillon 50 mg/ml de méthanol). Pour faciliter et accélérer la purification par HPLC des différents peptides de TCZ 11A, une préséparation a été réalisée sur le mélange TCZ 11A par chromatographie à pression normale, en utilisant une grande colonne de gel de silice (système éluant $CH_2Cl_2$ $-CH_3OH- H_2O$ 65/24/4). En opérant sur les fractions ainsi enrichies, des HPLC intensives avec le mélange éthanol-eau (65/35) comme éluant, de petites quantités de TCZ 11 AIII, TCZ 11 AIV, TCZ11 AVI, TCZ 11 AVII (peptides majoritaires) ont été isolées et une analyse structurale a été entreprise particulièrement pour TCZ 11 AIII, TCZ 11 A VI et TCZ 11 A VII. Toutes ces fractions font partie intégrante de l'invention.

La composition en acides aminés de ces peptides a été déterminée après hydrolyse totale par HCl 6 N à 110°C pendant 24 heures et 48 heures. Les compositions obtenues sont consignées dans le tableau III ci-après (P 38).

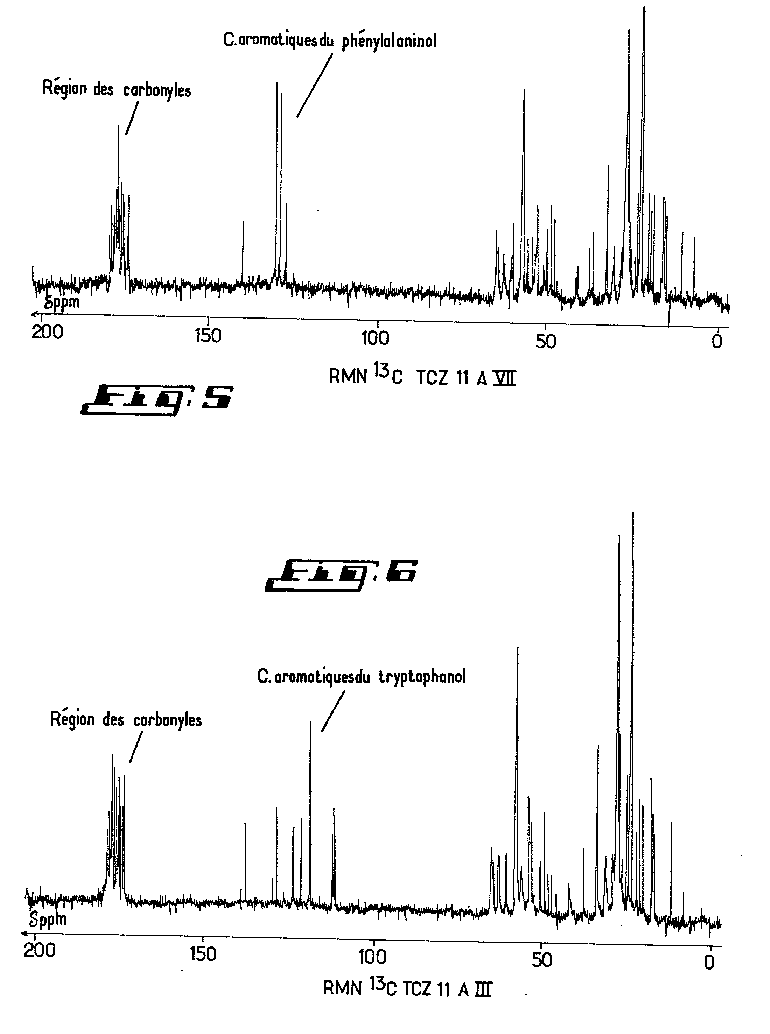

Des spectres RMN du proton et du carbone 13 ont été obtenus pour ces quatre peptides et montrent qu'ils peuvent se répartir en deux groupes selon qu'ils contiennent du tryptophanol (TCZ 11 AIII, TCZ 11 AIV) ou du phenylalaninol (TCZ 11 AVI et TCZ 11 A VII). Le spectre réalisé en RMN $^1$H à 80 MHz de la fraction VI du chromatogramme de la figure 2 dans $CD_3OD$ fait l'objet de la figure 3 et montre l'absence de tryptophanol.

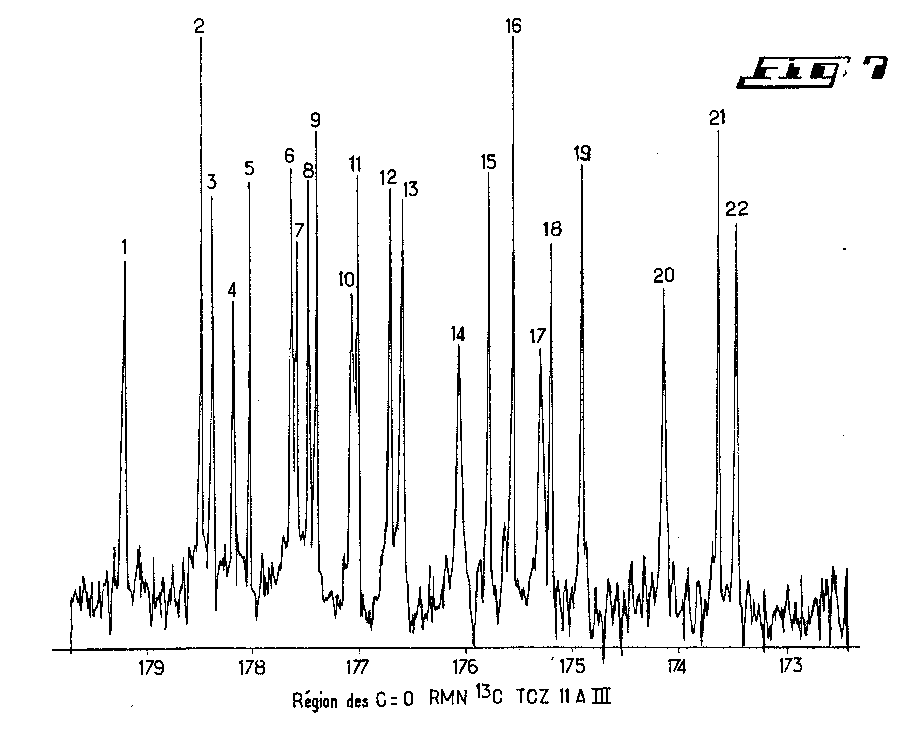

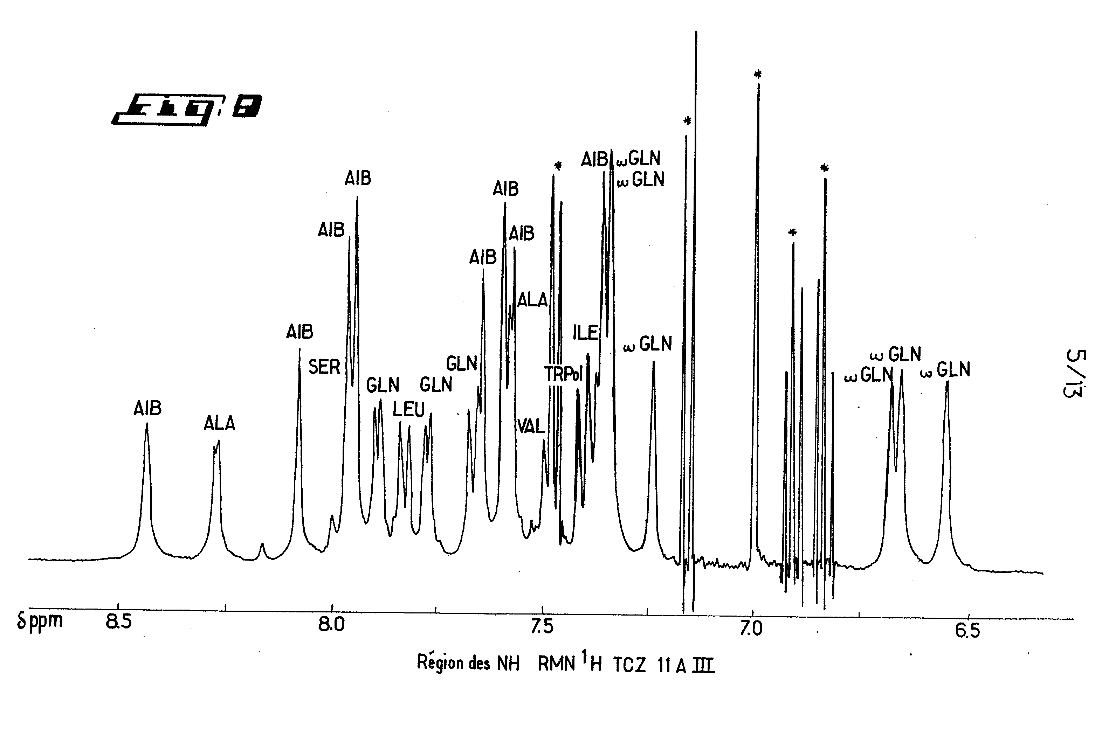

Le spectre RMN réalisé dans les mêmes conditions de la fraction IV fait l'objet de la figure 4 et celui de la fraction VII fait l'objet de la figure 5, ce dernier étant réalisé en RMN $^{13}$C à 20,1 MHz-$CD_3OD$ $^{12}$C..

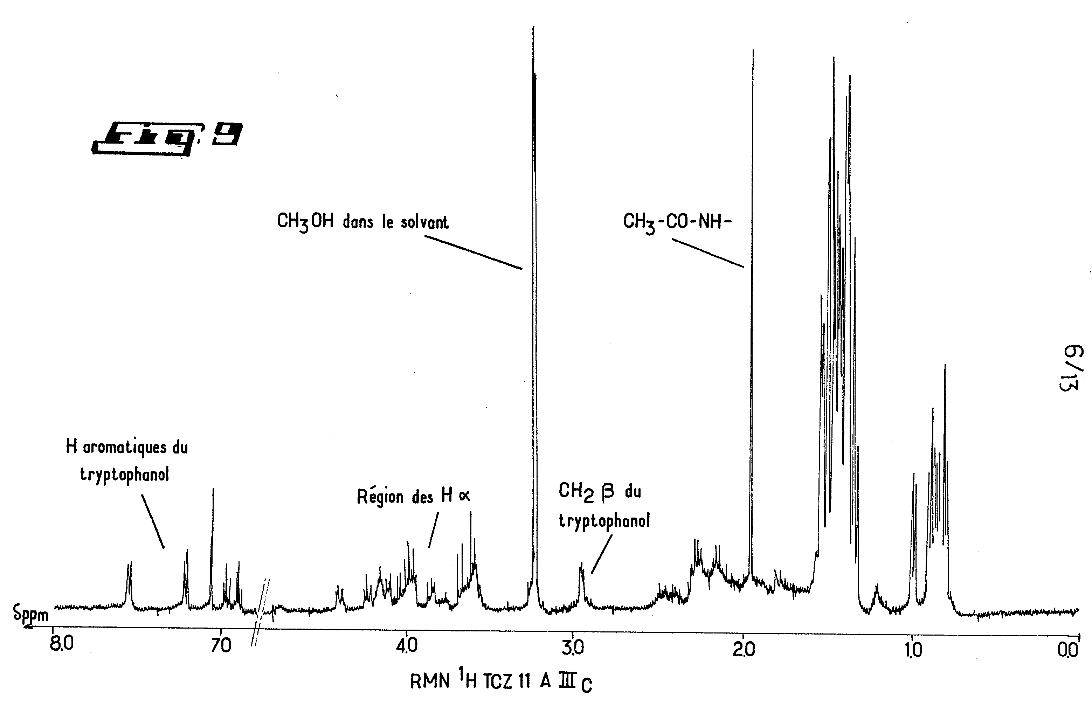

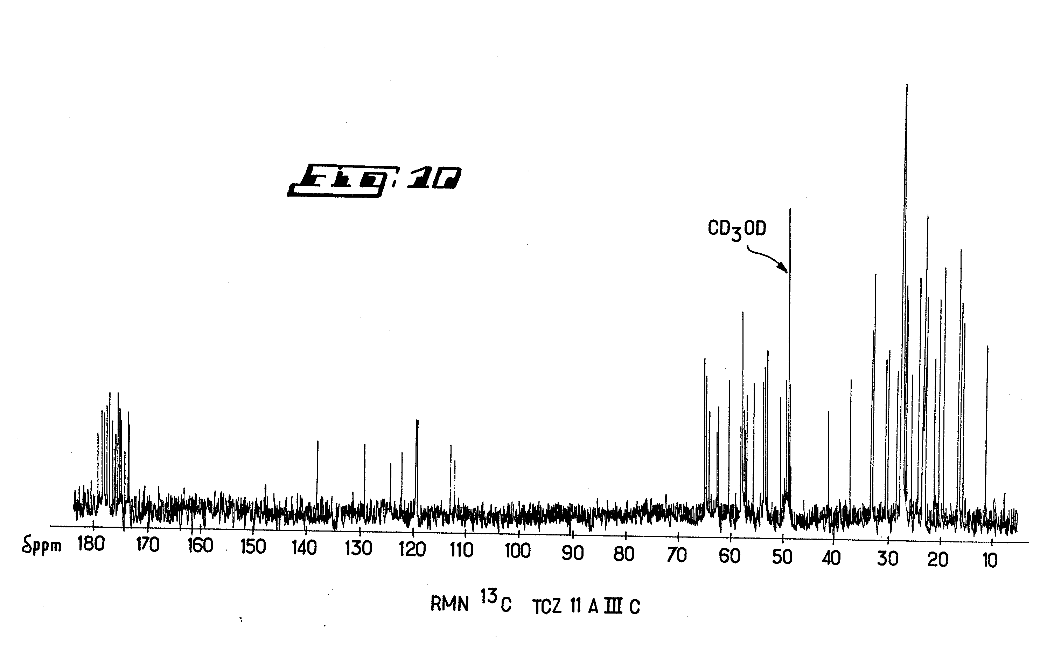

Enfin, la figure 6 concerne le spectre de RMN à 20,1 MHz en $^{13}$C et dans $CD_3OD$ $^{12}$C de la fraction III majoritaire ; la région des CO qui fait apparaître les 22 carbonyles de la molécule est mise en évidence sur la figure 7. La figure 8 concerne le spectre de RMN $^1$H de la fraction III majoritaire ou fraction TCZ 11 A III de la région des NH qui fait apparaître les fonctions NH de la molécule (conditions d'expérience 400 MHZ, dans $CD_3OH$).

La complexité anormale des signaux de RMN, ainsi que les valeurs quantitatives de l'hydrolyse peu satisfaisantes de ces peptides, amène à douter de la pureté des produits.

Les échantillons sont donc chromatographiés à nouveau par HPLC. Les conditions retenues sont : phase stationnaire $SiO_2$, éluant $CH_2-Cl_2$, EtOH 70/30 en rapport volumique.

Une analyse réalisée dans ces conditions montre que :

- TCZ 11 A III est décomposé en 2 peptides très minoritaires TCZ 11 A III A et TCZ 11 A III B et un peptide majoritaire TCZ 11 A III C ;

- que la fraction TCZ 11 A IV était décomposé en trois peptides : TCZ 11 A IV A ; TCZ 11 A IV B ; et TCZ 11 A IV C.

La fraction TCZ 11 A VI a été décomposé également en trois peptides : TCZ 11 A VI A ; TCZ 11 A VI B ; et TCZ 11 A VI C.

A la partie II du tableau donné en fin de description page 49 , on a récapitulé le schéma final de l'extraction des peptides ci-dessus.

En opérant à nouveau des chromatographies préparatives HPLC dans les nouvelles conditions ($SiO_2$ ; $CH_2Cl_2$ - ETOH) la Demanderesse a pu obtenir de petites quantités de Trichorzianines TCZ 11 A III C ; TCZ 11 A VI A, TCZ 11 A VI B.

La séquence des peptides ou Trichorzianines TCZ 11 A III C ; TCZ 11 A VI A ; TCZ 11 A VI B ; et TCZ 11 A VII a été déterminée en s'appuyant sur leurs spectres de masse et RMN à haut champ (500 MHz) et en utilisant une nouvelle technique de RMN à deux dimensions.

23

A - <u>Structure de la Trichorzianine TCZ 11 A III C</u>

La Trichorzianine TCZ 11 A III C est un solide incolore cristallisé fondant à 253-250°C optiquement actif
$( \alpha )_D^{22} = -23°$.

Sa composition en acides aminés a été déterminée
par chromatographie et analyse des produits de son
hydrolyse.

Le spectre d'absorption ultraviolette de ce peptide
donne ( $\lambda_{max}^{EtOH}$ = 284 nm, $\mathcal{E}$ = 5 400) et est
très proche de celui du tryptophane ( $\lambda_{max}^{EtOH}$ = 284 nm,
$\mathcal{E}$ = 5 500).

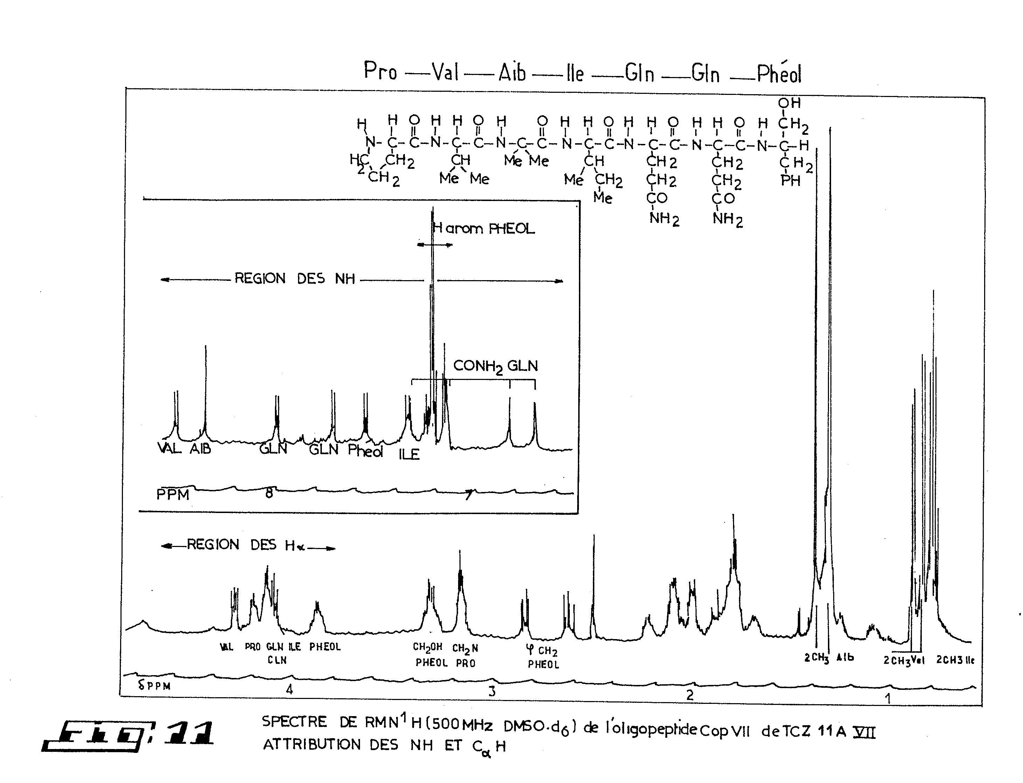

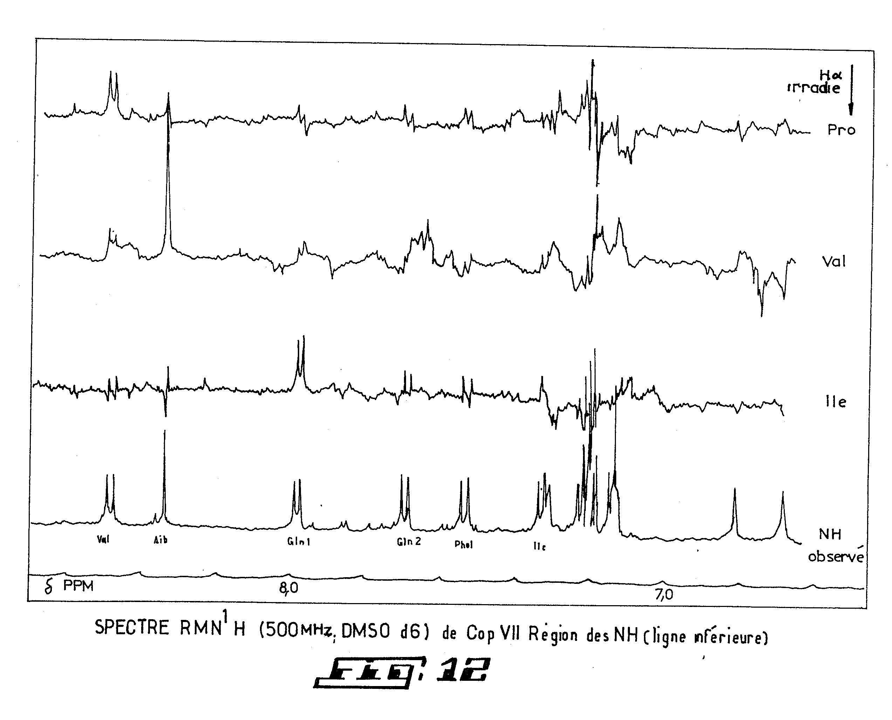

L'absence de réaction avec la ninhydrine a obligé à
la Demanderesse à vérifier la nature et le nombre des
aminoacides inclus dans la Trichorzianine TCZ 11 A III C
par une autre méthode : la résonnance magnétique nucléaire du proton (RMN $^1$H ) à haut champ (400 MHz), et
celle du carbone 13 (RMN $^{13}$C) à 100,6 MHz, qui font
l'objet respectivement des figures 9 et 10.

L'analyse de ces spectres de RMN de $^1$H et du $^{13}$C
de la Trichorzianine critallisée et purifiée dénommée
TCZ 11 A III C permet de la définir comme composée de :

- un groupe acétyle terminal $CH_3$-CO ;

- dix aminoacides possédant un CH $\alpha$ :

- 2Ala-1Val-1Leu-1Ile-Ser-1Pro-3Gln ;

- huit aminoacides dont le C est quaternaire :

(8AIB méthylalanine) ;

24

- un alcool aminé terminal : le tryptophanol.

La composition ainsi définie implique comme formule brute : $C_{91}H_{149}N_{23}O_{24}$ soit une masse moléculaire de 1 948 et un ion moléculaire à 1 947 daltons. L'analyse des principaux fragments est donné dans le tableau IV avec ceux des autres Trichorzianines. La partie de fragment principale Nop (de l'acétyle N terminal à la proline) donne des fragmentations qui sont désignées par N dans le tableau IV. La partie ou fragment Cop appa-raît à m/z 841 après capture d'un atome d'hydrogène et génère la famille d'ions-fragments signalés par C dans le tableau IV L'ensemble permet de proposer pour la Trichorzianine TCZ A III C les fragments de séquence complémentaires suivants :

Nop III : Ac Aib Ala Ala Aib Aib Gln Aib Aib Aib Ser ⎰Leu Aib
                                                       ⎱Ile

Cop III : Pro Val Aib ⎰Ile Gln Gln Trpol
                      ⎱Leu

Les valeurs des déplacements chimiques des protons dans le spectre de RMN $^{1}$H de TCZ A III C (solution dans le DMSO) sont consignées dans le tableau V.

Les mesures de RMN des effets Overhauser nucléaires ont permis de vérifier les hypothèses de séquence découlant de l'analyse du spectre de masse et de compléter ces résultats en permettant la distinction entre les amino-acides isobares (Leu-Ile).

Elles font apparaître les éléments de séquence sui-vants :

-Ile-Gln-Gln-Trpol

- Ser-Leu-
- Pro-Val-
- Ala-Ala-

Ces résultats permettent de lever l'ambiguïté Leu-Ile et de confirmer les positions-17 et -18 de deux glutamines.

| TCZ A IIIc | | | TCZA VII | | | TCZ A VIa | | | TCZ A VIb | | |
|---|---|---|---|---|---|---|---|---|---|---|---|
| m/n | Sér | % | m/n | Sér | % | m/n | Sér | % | m/n | Sér | % |
| <u>1108</u> | N | 17 | <u>1122</u> | N | 19 | <u>1122</u> | N | 7 | <u>1108</u> | N | 4 |
| 1023 | N | 1 | 1037 | N | 0,4 | | | | 1023 | N | 0,4 |
| 910 | N | 4 | 924 | N | 4 | 924 | N | 2 | 910 | N | 2 |
| <u>841</u> | C | 22 | | | | | | | | | |
| 823 | N | 11 | 837 | N | 19 | 837 | N | 10 | 823 | N | 9 |
| | | | <u>802</u> | C | 24 | <u>816</u> | C | 14 | <u>802</u> | C | 9 |
| 738 | N | 11 | 752 | N | 15 | 752 | N | 10 | 738 | N | 11 |
| 653 | N | 16 | 667 | N | 19 | 667 | N | 14 | 653 | N | 16 |
| 651 | C | 3 | 651 | C | 3 | 665 | C | 1 | 651 | C | 1 |
| 568 | N | 3 | 582 | N | 4 | 582 | N | 1,5 | 568 | N | 2 |
| 523 | C | 4 | 523 | C | 4 | 537 | C | 1,5 | 523 | C | 1 |
| 454 | | 12 | 454 | N | 81 | 454 | N | 37 | 454 | | 8 |
| 440 | N | 39 | 440 | | 6 | | | | 440 | N | 42 |
| 428 | | 23 | 410 | | 2 | 424 | | 1 | | | |
| 410 | | 5 | 408 | | 3 | 409 | C | 1 | 410 | | 1 |
| 395 | C | 6 | 395 | C | 4 | | | | 395 | C | 1 |
| 355 | N | 46 | 355 | N | 100 | 355 | N | 68 | 355 | N | 83 |
| 307 | | 39 | 313 | | 7 | | | | 299 | | 8 |
| 282 | C | 28 | 282 | C | 17 | 296 | C | 8 | 282 | C | 11 |
| 270 | N | 44 | 270 | N | 87 | 270 | N | 77 | 270 | N | 98 |
| 232 | | 43 | 228 | | 7 | 228 | | 7 | 242 | | 9 |
| 215 | | 60 | 214 | | 5 | | | | 228 | | 6 |
| 202 | | 55 | | | | 211 | C | 8 | 214 | | 7 |
| 199 | N | 70 | 199 | N | 56 | 199 | N | 100 | 199 | N | 100 |
| 197 | C | 44 | 197 | C | 8 | 155 | | 12 | 197 | C | 8 |
| 128 | N | 55 | 128 | N | 56 | 128 | N | 67 | 128 | N | 68 |
| 93 | | 100 | | | | 100 | | 56 | 100 | | 52 |

<u>Tableau IV</u>  -Principaux fragments observés dans les spectres de masse des Trichorzianines.Les deux familles de fragments dérivant des oligopeptides Nop et Cop sont désignées par les lettres N et C. Les intensités sont données en % du pic de base.

| $\delta$ ppm | allure (J Hz) | Attribution | $\delta$ ppm | allure (J Hz) | Attribution |
|---|---|---|---|---|---|
| 8,84 | s | NH indole | 4,760 | dd (5,5 et 7,0) | OH Ser |
| 8,539 | s | NH Aib | 4,597 | t (6,0) | OH Trpol |
| 8,242 | d (5,5) | NH Ala | 4,301 | m | $H_\alpha$ Leu |
| 7,946 | s | NH Aib | 4,262 | t (7,7) | $H_\alpha$ Pro |
| 7,847 | s | NH Aib | 4,123 | ddd (9,2-8,0-4,8) | $H_\alpha$ Gln-1 |
| 7,831 | s | NH Aib | 4,03 | m | $H_\alpha$ Ser |
| 7,785 | s | NH Aib | 4,03 | m | $H_\alpha$ Ala |
| 7,744 | d (4,8) | NH Ser | 4,03 | m | $H_\alpha$ Ala |
| 7,744 | d (5) | NH Gln-3 | 4,03 | m | $H_\alpha$ Gln-2 |
| 7,724 | d (6,4) | NH Ala | 3,952 | m | $H_\alpha$ Trpol |
| 7,698 | d(6,6) | NH Gln-2 | 3,919 | t (6,64) | $H_\alpha$ Ileu |
| 7,685 | d | NH Leu | 3,815 | t (7,3) | $H_\alpha$ Val |
| 7,685 | d | NH Val | 3,79 | m | $H_\alpha$ Gln-3 |
| 7,595 | m | Ar Trpol | 3,79 | m | $H_\beta$ Ser |
| 7,579 | s | NH Aib | 3,79 | m | $H_{\beta'}$ Ser |
| 7,541 | s | NH Aib | 3,700 | m | $H_\delta$ Pro |
| 7,517 | d (8,0) | NH Gln-1 | 3,562 | m | $H_\delta$ Pro |
| 7,472 | s | NH Aib | 3,415 | m | $CH_2O$ Trpol |
| 7,296 | dd | Ar Trpol | 3,350 | m | $CH_2O$ Trpol |
| 7,272 | d (8,4) | NH Trpol | 2,94 | | $CH_2Ar$ Trpol |
| 7,166 | d (7,2) | NH Ile | 2,76 | | $CH_2Ar$ Trpol |
| 7,146 | s | $2CONH_2$ Gln | 1,941 | s | $CH_3$ Ac |
| 7,127 | d (2,4) | Ar Trpol | 1,499 à 1350 | 16 s | $CH_3$ (8 Aib) |
| 7,081 | s | $CONH_2$ Gln | 1,350 | d (7,3) | $CH_3$ Ala |
| 7,032 | m | Ar Trpol | 1,324 | d (7,3) | $CH_3$ Ala |
| 6,951 | m | Ar Trpol | 0,964 | d (6,6) | $CH_3$ Val |
| 6,720 | s | $2CONH_2$ Gln | 0,902 | d (6,8) | $CH_3$ Val |
| 6,609 | s | $CONH_2$ Gln | 0,869 | d (6,8) | $CH_3$ Ile |
| | | | 0,847 | d (6,4) | $CH_3$ Leu |
| | | | 0,821 | t (7,4) | $CH_3$ Ile |
| | | | 0,785 | d (6,4) | $CH_3$ Leu |

Tableau V - Spectre de RMN $^1$H 5DMSO-$d_6$ ; 500,13 Mhz) de TCZ A III C
(s : singulet, d : doublet, t : triplet, m : multiplet)

28

La séquence proposée pour la nouvelle Trichorzianine de la présente invention TCZ A III C est donc la suivante :

Ac Aib Ala Ala Aib Aib Gln Aib Aib Aib Ser Leu Aib ProVal Aib Ile Gln Gln Trpol

### B - Structure de la Trichorzianine TCZ A VII

La composition en amino-acides de TCZ A VII a été définie comme : Aib (8), Gln (3), Ala (2), Val (1), Iva (1), Leu (1), Ile (1), Pro (1) et Ser (1), à la suite de l'analyse des acides aminés obtenus par hydrolyse totale et de l'examen du spectre de RMN $^1$H, le groupe N terminal est acétylé et le résidu C terminal est le phénylalaninol. Les groupes $CONH_2$, des trois glutamines ont été repérés dans le spectre de RMN [1]H.

Le spectre de masse de TCZ A VII fait apparaître l'ion moléculaire à m/z 1922 correspondant à la formule brute $C_{91}H_{152}N_{22}O_{24}$. Les principaux fragments observés (Tabl. IV col. 2) se répartissent en deux familles (notées N et C dans le tableau) et correspondent aux fragmentations des deux oligopeptides Nop VII et CopVII produits par la coupure au niveau de la proline. Ils permettent de proposer, pour ces deux oligopeptides, les séquences suivantes :

Nop VII : Ac Aib Ala Ala Aib⟨Iva Gln Aib Aib Aib Ser⟨Leu Aib
          ⟨Val              ⟨Ile

Cop VII : Pro⟨Val Aib⟨Ile Gln Gln Phéol
         ⟨Iva     ⟨Leu

Pour lever les ambiguïtés Val-Iva d'une part et Leu-

29

Ile, d'autre part, et pour vérifier la séquence de Cop VII, nous avons effectué une hydrolyse spécifique de TCZ 11 AVII au niveau de la proline et étudié les produits formés.

L'hydrolyse a été réalisée par l'acide trifluroacétique à 37°C pendant 24 h et les deux oligopeptides formés (Nop VII et Cop VII) ont été séparés par chromatographie.

| ppm | allure ( Hz) | | attribution | |
|---|---|---|---|---|
| 8,48 | d | (8,7) | NH | Val |
| 8,34 | s | | NH | Aib |
| 7,99 | d | (7,3) | NH | Gln-1 |
| 7,70 | d | (8,1) | NH | Gln-2 |
| 7,54 | d | (8,5) | NH | Phéol |
| 7,34 | d | (7,5) | NH | Ile |
| 7,32 | s | (large) | $CONH_2$ | Gln |
| 7,20 | massif | | Ar | Phéol |
| 7,15 | s | (large) | $CONH_2$ | Gln |
| 6,82 | s | (large) | $CONH_2$ | Gln |
| 6,69 | s | (large) | $CONH_2$ | Gln |
| 4,30 | dd | (8, 7-6,0) | H | Val |
| 4,20 | dd | (8,0-6,0) | H | Pro |
| 4,14 | m | | H | Gln |
| 4,14 | m | | H | Gln |
| 4,10 | dd | (7, 5-7, 0) | H | Ile |
| 3,88 | m | | H | Phéol |
| 3,31 | m | | $CH_2O$ | Phéol |
| 3,16 | m | | $CH_2$ | Pro |
| 2,84 | m | | $CH_2$ | Phéol |
| 2,62 | m | | $CH_2$ | Phéol |
| 2, 3-1, 4 | m | | | |
| 1,39 | s | | $CH_3$ | Aib |
| 1,33 | s | | $CH_3$ | Aib |
| 1,09 | m | | CH | Ile |
| 0,90 | d | (6,8) | $CH_3$ | Val |
| 0,85 | d | (6,8) | $CH_3$ | Val |
| 0,81 | d | (7,0) | $CH_3$ | Ile |
| 0,79 | t | (7,5) | $CH_3$ | Ile |

Tableau VI - Spectre de RMN $^1$H (DMSO-$d_6$ ; 500,13 MHz) de Cop VII.

Cop VII est aisément repéré en chromatographie sur couche mince : grâce à son résidu phénylalaninol, aromatique, il absorbe la lumière UV et donne une coloration jaune avec la ninhydrine puisque le résidu N terminal est la proline.

Son spectre de masse fait apparaître un ion pseudomoléculaire $(M+H)^+$ à m/z 802 est une fragmentation en accord avec la séquence proposée :

$$
\begin{array}{ccccccc}
& 197 & 282 & 395 & 523 & 651 & 801 \\
\text{Pro-} \Big\{ ^{\text{Val-}}_{\text{Iva}} & \text{Aib-} & \Big\{ ^{\text{Ile-}}_{\text{Leu}} & \text{Gln-} & \text{Gln-} & \text{Phéol}
\end{array}
$$

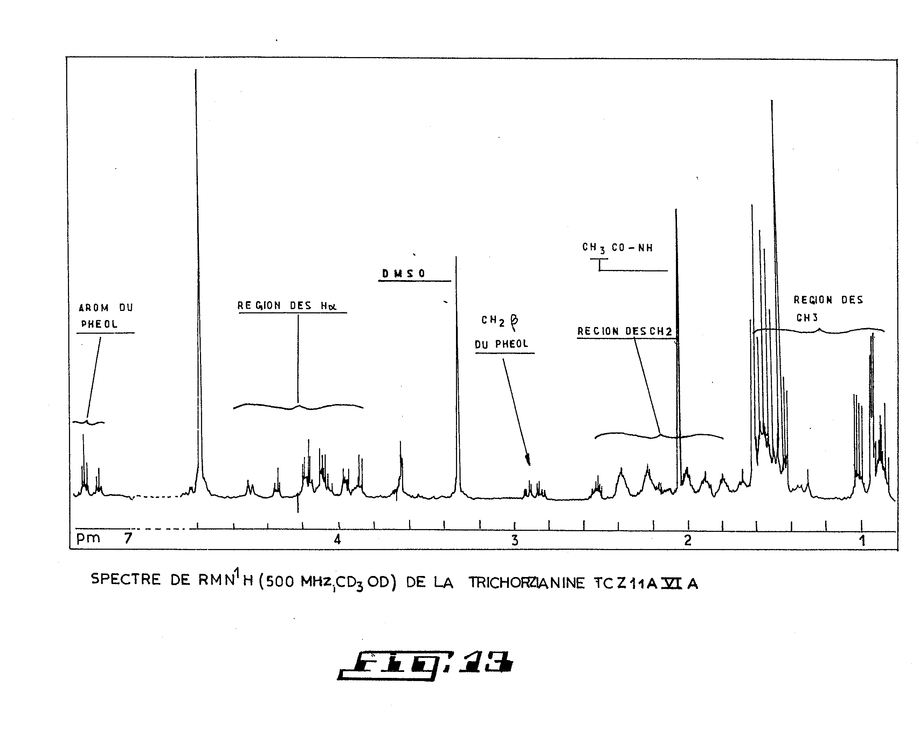

Le spectre de RMN $^1$H de Cop VII (cf. Tabl. VI et Fig. 11) montre la présence du Phéol, d'une Pro, d'un Aib, de 2 Gln, d'une Val et d'une Ile. Ces mêmes acides aminés sont repérés dans le chromatrogramme réalisé sur le produit de l'hydrolyse totale de Cop VII.

L'isovaline et la leucine identifiées dans la composition de TCZ 11 AVII ne sont pas retrouvées dans Cop VII et doivent, de ce fait, être situées dans l'oligopeptide complémentaire Nop VII.

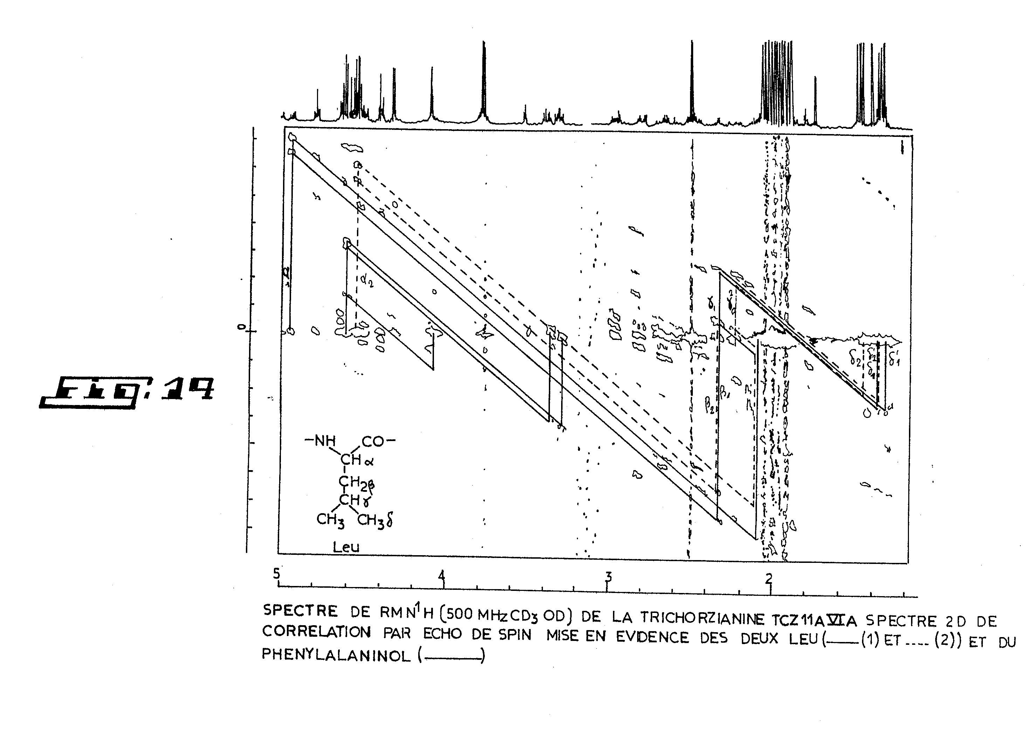

La mesure des effets NOE en RMN $^1$H (Fig. 12) pour Cop VII a permis de définir trois enchaînements :

Pro-Val-Aib-

Aib-Ile-Gln          et -Gln-Phéol

La séquence de Cop VII est donc :

Pro-Val-Aib-Ile-Gln-Gln-Phéol

Le fragment Nop VII est plus difficile à isoler et purifier. En effet, il ne possède aucun groupement réactif et n'absorbe pas la lumière UV étant constitué uniquement de résidus aliphatiques.

La seule méthode de détection qui se soit révélée efficace a consisté à pulvériser une solution d'anisaldéhyde et d'acide sulfurique dans l'acide acétique sur la plaque chromatographique. Après chauffage elle se colore en violet et Nop apparaît sous forme d'une tache blanchâtre.

Son analyse en spectrométrie de masse a confirmé la séquence proposée :

```
128 199 270 355 454 582 667 752 837
Ac Aib Ala Ala Aib Iva Gln Aib Aib Aib Ser Leu Aib
    1   2   3   4   5   6   7   8   9   10  11  12
```

L'ensemble de ces résultats permet de proposer pour TCZ 11 A VII la séquence suivante :

Ac Aib Ala Ala Aib Iva Gln Aib Aib Aib Ser Leu Aib Pro Val Aib Ile Gln Gln Phéol

C - Structure_de_la_trichorzianine_TCZ_11_A_VI_A

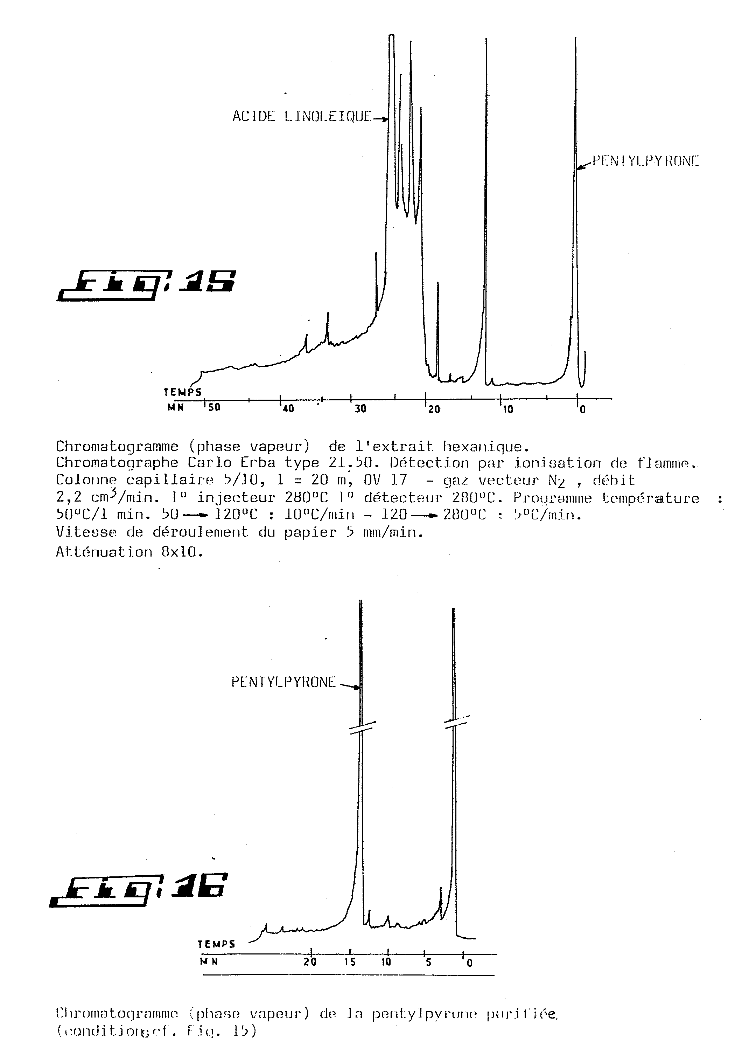

Le spectre de RMN $^1$H (cf. Tabl. VII et Fig. 13 de ce peptide met en évidence 18 amino-acides :

Aib (7), Ala (2), Leu (2), Ile ( 1), Iva (1), Pro (1), Ser (1) et Gln (3).

Il fait apparaître la présence d'un groupe acétyle ($CH_3CO$) fixé sur le résidu N terminal et celle d'un résidu phénylalaninol.

33

En fait, le spectre est très semblable à celui de TCZ 11 A VII à deux exceptions près : le résidu Val n'est pas retrouvé et un deuxième résidu Leu est observé (Fig. 14).

La spectrométrie de masse définit l'ion moléculaire à m/z 1936 (formule brute : $C_{91}H_{152}N_{22}O_{24}$).

La séquence de TCZ 11 A VI A peut être déduite de l'examen des fragmentations en spectrométrie de masse (Tabl. IV, col. 3).

En effet, on retrouve à nouveau les familles de fragments Nop et Cop déjà signalées.

La famille Nop VIa est identique à celle de Nop VII observée dans la fragmentation de la Trichorzianine TCZ 11 A VII et est initiée à m/z 1122. Nop VIa est donc identique à Nop VII.

| ppm | allure ( Hz) | | attribution | |
|---|---|---|---|---|
| 4,482 | dd | (3,6-11,5) | H | Leu-1 |
| 4,335 | t | (8,1) | H | Pro |
| 4,188 | dd | (3,2-6,7) | H | Ser |
| 4,176 | dd | (6,0-9,0) | H | Gln-1 |
| 4,166 | q | (7,3) | H | Ala-1 |
| 4,131 | m | (5,3) | H | Phéol |
| 4,089 | dd | (4,9-10,6 | H | Leu-2 |
| 4,086 | q | (7,4) | H | Ala-2 |
| 4,062 | t | (7,5) | H | Gln-2 |
| 4,043 | dd | (6,8-11,5) | H | Ser |
| 3,946 | dd | (6,5-8,7) | H | Gln-3 |
| 3,945 | dd | (3,2-11,5) | H | Ser |
| 3,883 | m | | H | Pro |
| 3,864 | d | (8,1) | H | Ile |
| 3,650 | m | | H | Pro |
| 3,661 | ddd | | $CH_2O$ | Phéol |
| 2,90 | dd | | Ar $CH_2$ | Phéol |
| 2,84 | dd | | Ar $CH_2$ | Phéol |
| 1,621-1,466 | | 15s | $CH_3$ | 7Aib |
| | | | $CH_3$ | Iva |
| 1,473 | d | (7,3) | $CH_3$ | Ala-1 |
| 1,429 | d | (7,4) | $CH_3$ | Ala-2 |
| 1,335 | m | | H | Ile |
| 1,020 | d | (6,5) | $CH_3$ | Leu-2 |
| 0,994 | d | (6,8) | $CH_3$ | Ile |
| 0,932 | d | (6,5) | $CH_3$ | Leu-1 |
| 0,926 | d | (6,5) | $CH_3$ | Leu-2 |
| 0,926 | t | (7,3) | $CH_3$ | Ile |
| 0,889 | d | (6,4) | $CH_3$ | Leu-1 |
| 0,858 | t | (7,5) | $CH_3$ | Iva |

Tableau VII- Spectre de RMN $^1$H (500,13 MHz ; $CD_3OD$) de TCZ 11 A VI A

Nop VIa : Ac Aib Ala Ala Aib Iva Gln Aib Aib Aib Ser Leu Aib

La famille Cop VIa initiée à m/z 816 est décalée de +14 unités de masse pour l'ensemble des fragments observés par rapport à ceux de Cop VII. Ceci est expliqué par le remplacement de la valine de Cop VII par la leucine supplémentaire dans le cas de Cop VIa, en accord avec les résultats de la RMN. La séquence de Cop VIa est donc :

211 286 409 537 665     816
Pro Leu Aib Ile Gln Gln Phéol + H$^+$

Ces résultats conduisent à proposer pour TCZ 11 A VI A la séquence :

Ac Aib Ala Ala Aib Iva Gln Aib Aib Aib Ser Leu Aib Pro Leu Aib Ile Gln Gln Phéol

Signalons que les résultats acquis actuellement ne permettent pas de prouver de façon rigoureuse les positions des acides aminés isobares Leu-Ile qui pourraient être intervertis éventuellement.

Cependant la grande similitude des déplacements chimiques des protons dans les spectres de RMN $^1$H de TCZ 11 A VI A et TCZ 11 A VII laisse supposer une structure très apparentée.

Par ailleurs, l'enchaînement Ile-Gln Gln devra être vérifié.

D- Structure de la trichorzianine TCZ 11 A VI B

La structure proposée pour ce peptide découle pour l'essentiel de l'analyse de son spectre de masse (ionisa-

36

tion "Fab" positive). Il fait apparaître l'ion à m/z 1908 correspondant à la formule brute $C_{89}H_{148}N_{22}O_{24}$.

La famille d'ions Nop VIb commence à 1108 unités de masse atomique et est identique à celle observée pour Nop III dans le spectre de TCZ 11 A III C (Tabl. IV col.4). Ceci conduit à attribuer la même séquence pour cette partie de la molécule :Nop VIb ≡ Nop III

Ac Aib Ala Ala Aib Aib Gln Aib Aib Aib Ser Leu Aib

La famille d'ions Cop VIb, initiée à m/z 802 après capture d'un atome d'hydrogène est identique à celle déjà décrite pour Cop VII. La séquence de ce fragment est donc :

Cop VIb ≡ Cop VII :

Pro Val Aib Ile Gln Gln Phéol

En gardant les mêmes réserves quant à la position réciproque des acides aminés isobares que dans le cas précédent (TCZ 11 A VI A) ces résultats conduisent à proposer pour TCZ 11 A VI B la séquence :

Ac Aib Ala Ala Aib Aib Gln Aib Aib Aib Ser Leu Aib Pro Val Aib Ile Gln Gln Phéol

En conclusion, les résultats acquis montrent une grande similitude de structures entre les trichorzianines identifiées jusqu'à présent : TCZ 11 A VIA ne diffère de TCZ 11 A VII que par un seul groupe $CH_2$ (changement d'une valine en leucine) et il en est de même pour TCZ 11 A VI B par rapport à TCZ 11 A VII (changement d'une méthylalanine (Aib) en éthylalanine (Iva)) et par rapport à TCZ 11 A III C (remplacement de Trpol en Phéol).

Ces faibles différences de structure expliquent les difficultés rencontrées au cours des séparations chromatographiques. Rappelons enfin que les trichorzianines B diffèrent des trichorzianines A par la présence d'acides glutamiques.Il est à noter encore une fois que toutes les Trichorzianines sont nouvelles et font partie intégrante de l'invention. En outre elles sont toutes actives et sont donc selon l'invention utiliées ou appliquées comme moyen de lutte biologique, en particulier sous forme de produit phyto-sanitaire.

TABLEAU III COMPOSITION EN ACIDES AMINES DES PEPTIDES (TRICHORZIANINES) DE TRICHODERMA HARZIANUM I 223, ATCC N°20672

| | Glycine | Alanine | Méthyl-Alanine | Valine | Iso-valine | Leucine | Iso-leucine | Proline | Hydroxy-proline | Acide gluta-mique | Glycine | Phényl-alanine | Trypto-phane | Sérine | Thréo-nine | |
|---|---|---|---|---|---|---|---|---|---|---|---|---|---|---|---|---|
| ALAMETHICINE | 1 | 2 | 8 | 2 | 0 | 1 | 0 | 2 | 0 | 1 | 2 | 0 | 0 | 0 | 0 | Phènyl-alaninol |
| SUKUKACILLINE | 1 | 2 | 10 | 3 | 0 | 2 | 0 | 2 | 0 | 1 | 2 | 0 | 0 | 0 | 0 | Phényl-alaninol |
| TRICHOTOXINE | 1 | 2 | 9 | 0 | 1 | 1 | 0 | 1 | 0 | 0 | 2 | 0 | 0 | 0 | 0 | Valinol |
| HYPELCINE A | 1 | 1 | 10 | 1 | 0 | 1 | 0 | 2 | 0 | 0 | 3 | 0 | 0 | 0 | 0 | Leucinol |
| EMERICINE III | 1 | 1 | 5 | 1 | 1 | 1 | 0 | 0 | 2 | 0 | 1 | 1 | 0 | 0 | 0 | Phényl-alaninol |
| ANTIAMOBEINE | 1 | 0 | 6 | 0 | 2 | 1 | 0 | 1 | 2 | 1 | 1 | 1 | 0 | 0 | 0 | Phényl-alaninol |
| ZERVAMICINE 1A | 0 | 0 | 4 | 1 | 1 | 1 | 1 | 1 | 2 | 1 | 1 | 0 | 1 | 0 | 1 | Phényl-alaninol |
| ZERVAMICINE IIB | 0 | 0 | 4 | 0 | 1 | 1 | 2 | 1 | 2 | 0 | 2 | 0 | 1 | 0 | 1 | alaninol |
| Peptides isolés de Trichoderma I223 ou ATCC ou Trichorzianines | | | | | | | | | | | | | | | | |
| III | 0 | 3 | 7,2 | 2 | | 1,2 | 0,8 | 1 | 0 | 3,6 | | 0 | 1 | 1 | 0 | Tryptophanol |
| IV | 0 | 3 | 7 | 2,8 | | 1,2 | 0,8 | 1 | 0 | 3,6 | | 0 | 1 | 1 | 0 | Tryptophanol |
| VI | 0 | 3 | 6,6 | 3 | | 1,2 | 0,8 | 1 | 0 | 3,6 | | 0 | 0 | 1 | 0 | Phényl-alaninol |
| VII | 0 | 3 | 6,6 | 3 | | 1,2 | 0,8 | 1 | 0 | 3,6 | | 0, | 0 | 1 | 0 | Phényl-alaninol |

Le peptide majoritaire est le peptide III

En outre, des analyses des extraits peptidiques bruts ou Trichorzianines obtenus à partir de différents clones, c'est-à-dire obtenus à partir d'une souche unique de Trichorderma harzianum I 223 ou ATCC n° 20672 ont montré une identité pour toutes les souches ce qui démontre que la multiplicité des peptides est une caractéristique génétique. La teneur en peptides est variable selon les conditions de culture et celle-ci peut atteindre 2 % en poids de peptides ou Trichorzianines par poids de produit final, sur milieu riche.

On donne ci-après un exemple d'essai de pouvoir antagoniste.

EXEMPLE 6

Essai de pouvoir antagoniste de Trichorderma harzianum I 223 ou ATCC n° 20672

Cet essai de pouvoir antagoniste est effectué in vitro en boîte de Pétri.

Une goutte de suspension contenant $10^6$ spores vivantes de Trichorderma harzianum I 223 ou ATCC n° 20672 est placée au bord d'une boîte de Pétri sur le milieu gélosé de croissance du Botrytis cinerea, en l'occurence ce milieu est essentiellement constitué de concentré de tomate.

A l'opposé, on place une pastille de 4 millimètres prélevée dans une boîte de Pétri où l'on a réalisé la croissance du Botrytis cinerea.

Après trois jours d'incubation, la croissance du champignon parasite est freinée par rapport à la crois-

40

sance témoin de ce même champignon et après cinq jours, la croissance de Trichoderma harzianum I 223 ou ATCC n° 20672 a rejoint le Botrytis cinerea et commence à le recouvrir progressivement.

On constate donc ici le fort pouvoir antagoniste par contact de Trichoderma harzianum I 223 ou ATCC n° 20672 vis-à-vis du chmapignon parasite.

Le même essai a été réalisé à l'égard d'autres champignons parasites tels que Stereum purpureum, Sclerotinia, Stereum hirsutum et Ceratocystis ulmi. Tous ces essais se sont révélés positifs avec la nouvelle souche de Trichoderma harzianum I 223 ou ATCC n° 20672.

Ce même essai de pouvoir antagoniste par contact a été réalisé également sur tous ces champignons parasites pour les peptides extraits de Trichoderma. Ceci fait l'objet de l'exemple 8.

EXEMPLE 7

Pouvoir antagoniste à distance de Trichoderma harzianum I 223 ou ATCC n° 20672

Pour réaliser cet essai de pouvoir antagoniste à distance de Trichoderma harzianum I 223 ou ATTC n° 20672 on inhibe tout d'abord la croissance du mycélium de Trichoderma harzianum I 223 ou ATCC n° 20672 par une barrière de fongicide chimique connu pour inhiber la croissance de Trichoderma harzianum sans inhiber celle du parasite, comme par exemple le Bénomyl et on place à l'opposé une pastille de 4 millimètres prélevée dans une boîte de Pétri où l'on a réalisé la croissance du parasite.

41

On observe si la croissance du mycélium du champignon parasite est freinée.

L'évaluation de ce ralentissement de croissance permet d'apprécier la production et la diffusion des polypeptides antifongiques ou Trichorzianines sécrétés par la souche de Trichoderma harzianum I 223 ou ATCC n° 20672.

La Demanderesse a effectué un essai de pouvoir antagoniste à distance pour chacun des champignons parasites mentionnés précédemment et a pu observer que la nouvelle souche de Trichoderma harzianum I 223 ou ATCC n° 20672 avait un fort pouvoir antagoniste à distance relativement à chacun de ces parasites.

EXEMPLE 8

Pouvoir antagoniste des Trichorzianines ou peptides extraits de Trichoderma harzianum I 223 ou ATCC n° 20672

L'extrait de Trichorzianine est mélangé à un milieu gélosé dans une boîte de Pétri. Lorsque le milieu est refroidi, un inoculum dense des différents parasites à tester est placé au centre de la boîte.

On laisse incuber à la température optimale pour les parasites, on constate un ralentissement de la croissance du parasite ou même une inhibition complète et ceci démontre le pouvoir antagoniste des Trichorzianines vis-à-vis des champignons parasites tels que Botrytis cinerea, Stereum purpureum, Sclerotinia, Stereum hirsutum ou Ceratocystis ulmi.

42

EXEMPLE 9

Préparation de produit phytosanitaire

On obtient une préparation de produit phytosanitaire en mettant en suspension dans l'eau, par exemple l'eau du robinet, la poudre obtenue selon l'un quelconque des ecemples 1 à 4, et de préférence obtenue à partir de l'exemple 3 ou de l'exemple 4, en vue de préférence à obtenir une concentration de $10^8$ spores viables/ml de la solution.

Cette solution en suspension est préparée de préférence juste avant l'application par pulvérisation sur le milieu à protéger, qui peut être des plants de vigne (lutte contre Botrytis cinerea, cause de la pourriture grise de la vigne) ou des arbres fruitiers (lutte contre Stereum purpureum responsable de la maladie du plomb des arbres fruitiers) ou de l'orme (lutte contre Ceratocystis ulmi responsable de la graphiose de l'orme).

Cette pulvérisation peut être répétée autant que nécessaire de manière habituelle jusqu'à guérison complète.

EXEMPLE 10

Traitement de la souche de Trichoderma harzianum I 223 ou ATCC n° 20672 pour la rendre résistante aux antifongiques chimiques

Il est préféré selon l'invention de modifier la souche de Trichoderma harzianum I 223 ou ATCC n° 20672 pour la rendre résistante aux agents chimiques en vue d'une utilisation du produit en traitement de lutte

43

intégrée, c'est-à-dire un traitement avec antifongiques chimiques plus traitement avec souche de Trichoderma.

Pour ce faire, il est nécessaire de rendre la souche résistante aux antifongiques chimiques (lorsqu'elle ne l'est pas, ce qui est également le cas) en particulier : bénomyl, procymidone, iprodione, peltar, etc.

Pour cela, on procède à une adaptation de la souche de Trichoderma harzianum I 223 ou ATCC n° 20672 à des quantités croissantes d'antifongiques chimiques et on procède comme suit :

Sur un milieu gélosé à base de flocons d'avoine, on ajoute une quantité d'antifongiques chimiques, légèrement inférieure à la quantité minimale inhibitrice déterminée comme indiqué plus loin.

On étale une suspension de spores de Trichoderma harzianum I 223 et l'on incube à 24°C sous lumière.

Après une incubation plus longue que la normale (supérieure ou égale à 5 jours), quelques colonies apparaissent.

Ces colonies sont repiquées sur le milieu gélosé aux flocons d'avoine contenant une dose supérieure ou égale à la dose précédemment employée de l'antifongique et ainsi de suite jusqu'à obtention d'une résistance aux quantités habituelles utilisées dans les champs.

Des quantités minimales inhibitrices sont préalablement déterminées pour chaque antifongique chimique en observant la croissance de la souche sur le milieu

44

aux flocons d'avoine auquel on a ajouté des quantités croissantes d'antifongique chimique.

Lorsque la souche cesse de croître, la quantité minimale inhibitrice d'antifongique chimique est atteinte.

Pour le bénomyl, cette quantité minimale inhibitrice est de 0,1 $\gamma$ /ml de gélose.

Ainsi, selon la présente invention, lorsque la souche de Trichoderma harzianum I 223 ou ATCC n° 20672 est utilisée comme moyen de lutte biologique, alors, de préférence cette souche est rendue résistante aux agents chimiques conformément à l'essai précédent.

EXEMPLE 11

Exemple d'extraction de pentyl-6 pyrone-2 à partir des spores de Trichoderma harzianum I 223 ou ATCC n° 20672

On a vu lors du procédé général d'extraction des spores produites par Trichoderma harzianum I 223 ou ATCC n° 20672 pour en isoler les peptides que la fraction soluble dans l'éther était active contre des champignons parasites tels que Botrytis cinerea dont on a indiqué que la substance principale responsable de cette activité a été isolée et identifiée par l'examen de ses spectres de RMN et de masse comme s'agissant de la pentyl-6 pyrone-2.

Le présent exemple est relatif à une méthode d'extraction des spores permettant l'extraction sélective de la pentyl-6 pyrone-2 sans les Trichorzianines. La technique la plus efficace consiste à une extraction par l'hexane et est de manière générale la suivante,

4 5

cette méthode faisant partie intégrante de la présente invention.

Dans une première étape, on introduit les spores de culture dans de l'hexane pour y dissoudre la partie soluble des spores et obtenir après évaporation de l'hexane un résidu huileux ;

On soumet ensuite ce résidu huileux à une étape d'extraction par l'acétone ou le chlorure de méthylène pour en recueillir la fraction soluble.

Enfin, après évaporation de l'acétone ou du chlorure de méthylène, on obtient une huile orangée se gélifiant par refroidissement et qui contient la pentyl-6 pyrone-2 en mélange notamment avec de l'acide linoléique.

De préférence, on soumet ce mélange à une chromatographie sur colonne de silice avec pour éluant un mélange de pentane avec des quantités croissantes d'éther.

Selon une variante de séparation de ce mélange, on réalise une chromatographie en phase vapeur.

Ce procédé général est maintenant exemplifié comme suit :

On extrait 987 g de spores , de préférence obtenues à partir de l'un quelconque des exemples 1 à 4, deux fois consécutivement par 3 litres d'hexane (RP redistillé) pendant 24 heures sous agitation. La solution hexanique évaporée à sec fournit un résidu huileux. Celui-ci est repris par 150 cm$^3$ d'acétone, et laisse déposer 1,9 g d'un solide blanc cristallisé identifié comme le palmitate d'ergostérol.

46

Par évaporation de l'acétone on obtient 13,3 g (1,35 %) d'une huile orangée se gélifiant par refroidissement (fraction H).

A - <u>Analyse par chromatographie en phase vapeur</u>

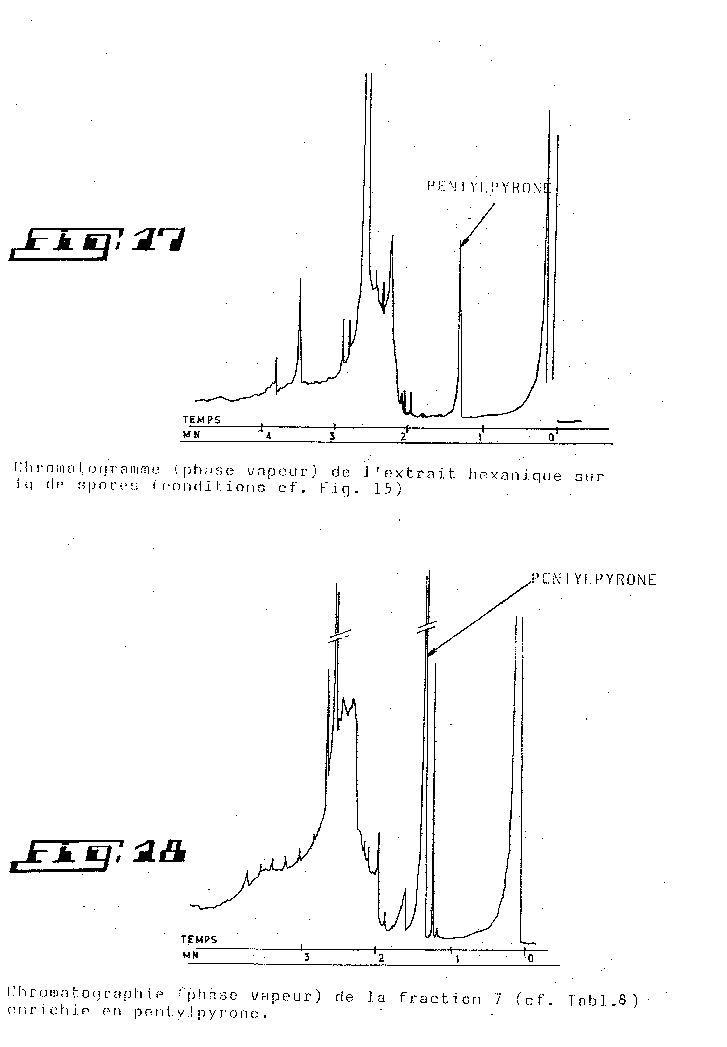

La fraction H, analysée par chromatographie en phase vapeur, montre une série de pics (Figure 15) parmi lesquels deux ont été identifiés par comparaison avec des échantillons de référence : l'un de temps de rétention, t = 12,2 minutes, est la pentyl-6 pyrone-2, l'autre (t = 25,4 minutes) est l'acide linoléique (Figure 16). La figure montre que le pic de la pentyl-6 pyrone-2 est nettement séparé des autres et que la méthode peut être employée pour doser ce composé.

Dans ce but, la Demanderesse a défini un protocole : il consiste à extraire 1 g de spores pendant 24 heures par 50 ml d'hexane sous agitation. La solution hexanique est filtrée pour éliminer les spores et le filtrat évaporé à sec. Le résidu (environ 15 mg) est dissous dans 1 ml de chlorure de méthylène. L'injection de 2 microlitres de cette solution donne le chromatogramme présenté figure 17. Ce chromatogramme fait apparaître que la pentyl-6 pyrone-2 est minoritaire dans l'extrait et, pour augmenter la précision du dosage, la Demanderesse a essayé de préparer un extrait enrichi.

B - <u>Préparation d'une fraction enrichie en pentyl-6 pyrone-2</u>

La méthode consiste à opérer une chromatographie sur colonne de silice en utilisant comme éluant un mélange de pentane avec des quantités croissantes d'éther. On opère sur 1 g de mélange et on collecte des fractions de 50 ml qui sont évaporées.

4 7

La pentyl-6 pyrone-2 apparaît dans la fraction 7 dont elle est un des constituants prépondérants, comme le montre le chromatogramme de la figure 18. La fraction 6 est essentiellement constituée d'acide linoléique.

Le bilan de la chromatographie sur gel de silice de l'extrait hexanique est donné au tableau suivant (tableau VIII).

| Solvant pentane/éther | Quantité (ml) | N° Fraction collectée | Poids du résidu (mg) | Observ. |
|---|---|---|---|---|
| 100/0 | 100 | 4 | 0 | |
| | | 5 | 190 | |
| 90/10 | 100 | 6 | 480 | acide linoléique |
| 50/50 | 100 | 7 | 150 | fraction contenant la pyrone |
| | | 8 | 50 | |
| 10/90 | 100 | 9 | 10 | |
| | | 10 | 10 | |
| 0/100 | 400 | 11 | 0 | |

Tableau VIII - Bilan de la chromatographie (SiO$_2$) de l'extrait hexanique.

4 8

En conclusion de ce qui précède, on peut donc extraire la pentyl-6 pyrone-2 sélectivement à partir des spores de Trichoderma harzianum I 223 ou ATCC n° 20672 par une extraction à l'hexane suivie d'une étape d'extraction dans l'acétone ou le chlorure de méthylène avec une séparation soit directement par chromatographie en phase vapeur (de préférence on utilise alors le chlorure de méthylène) ou après évaporation du solvant constitué par l'acétone ou le chlorure de méthylène, on réalise une séparation du mélange du produit gélifié par chromatographie sur colonne de silice avec comme éluant un mélange de pentane avec des quantités croissantes d'éther en recueillant la fraction 7 apparaissant entre 12 et 15 minutes après l'injection comme cela ressort du chromatogramme de la figure 18.

EXEMPLE 12

Mesure de l'activite spécifique de la pentyl-6 pyrone-2

En procédant comme décrit aux exemples 6 et 7, on a pu déterminer que la pentyl-6 pyrone-2 provoque une nette inhibition de la croissance de champignons parasites et en particulier de Penicillium crustosum, Botrytis cinerea.

Compte tenu de ces résultats, la Demanderesse a démontré que la pentyl-6 pyrone-2 a un pouvoir antagoniste relativement aux champignons parasites et en particulier Penicillium crustosum et Botrytis cinerea et peut donc être utilisée comme moyen de lutte biologique, en particulier sous forme de produit Phytosanitaire.

TABLEAU IX

PARTIE I
━━━━━━━━

FRACTION HAUT POIDS MOLECULAIRE
ISSUE DE LA FILTRATION SUR GEL LH 20

Chromatographie colonne $SiO_2$ ($CH_2Cl_2$/MeOH/$H_2O$ : 65/24/4)

TCZ 13     TCZ 11 A     TCZ 11 A + TCZ 11 B     TCZ 11B+Lécithine

Chromatographie
$Al_2O_3$ : (EtOH)

Chromatographie
(MeCOMe/MeOH   80/20→50/50 )

TCZ 11 A

TCZ 11 B   Lécithine

PARTIE II
━━━━━━━━━

Chromatographie HPLC $SiO_2$-$C_{18}$   (EtOH/$H_2O$ : 65/35)

TCZ 11 A III     TCZ 11 A IV     TCZ 11 A VI     TCZ 11 A VII

Chromatographie HPCL $SiO_2$ ($CH_2Cl_2$/EtOH : 70/30)

TCZ11 AIIIa   TCZ11 AIIIb   TCZ11 AIIIc   TCZ11 AIVa   TCZ11 AIVb   TCZ11 AIVc   TCZ11 AVIa   TCZ11 AVIb   TCZ11 AVIc

Protocole expérimental de séparation des trichorzianines à
partir de la fraction peptidique extraite des spores de Trichoderma
harzianum.
(TCZ:Trichorzianine, MeOH : méthanol, MeCOMe : acétone, SiO2 : gel
de silice, $SiO_2$_ $C_{18}$: gel de silice greffée $C_{18}$).

50

Revendications

1. Nouvelle souche de Trichoderma harzianum,
caractérisée en ce qu'il s'agit de la souche
Trichoderma harzianum déposée à l'Institut Pasteur
sous le N° I223 ou à l'ATCC sous le N° 20672.

2. Nouvelle souche de Trichoderma harzianum selon la
revendication 1, caractérisée en ce qu'elle présente
l'aspect cultural suivant :

- un mycélium ras, translucide à peine discernable
à l'oeil nu, à la surface duquel apparaissent au
bout de trois jours environ de culture, des formations
sporogènes d'abord blanches qui se colorent par la
suite en vert sombre;

- structure sporifère :

les conidiophores portent des branches ramifiées de
façon orthogonale, les cellules sporogènes (phialides) portées par ces dernières peuvent être groupées
par deux ou trois, elles sont de taille variable
(8 à 12 microns) étroites (2,5 microns) peu ventrues
et souvent recourbées, les spores qu'elles portent
sont globuleuses à sub-globuleuses, colorées en vert
et mesurent en moyenne 3 microns de diamètre, elles
apparaissent presque lisses au grandissement 1000;

La paroi des spores est complètement lisse au
microscope;

- la température de croissance est de 17 à 32°C avec
un optimum de 20 à 24°C;

- elles poussent sur milieu classique :

* malt-agar
* pomme de terre-glucose-agar,
* flocons d'avoine

et à un pH compris entre 4 et 7 et avec un optimum vers 5

La sporulation est plus importante sur un milieu riche;

- sa croissance est aérobie.

3. Procédé d'isolement de la nouvelle souche de Trichoderma harzianum I223 ou ATCC N° 20672, caractérisé en ce qu'il comprend les étapes successives suivantes :

A - on prélève des champignons de Trichoderma harzianum dans un endroit approprié tel que sol humide comme un sol maraîcher ou un sol cultivé;

B - on fait croître les champignons de Trichoderma harzianum prélevés sur un milieu de culture gélosé en boîte de Pétri à 24°C à la lumière pendant 3 à 7 jours;

C - on prélève le maximum de spores de Trichoderma harzianum de couleur verte;

D - on met en suspension dans l'eau ce prélèvement de Trichoderma harzianum pour dissocier les spores;

E - on détermine la ou les meilleures souches parmi les souches dissociées par un programme de sélection par clonage de préférence réalisé comme suit :

a) on effectue une culture séparée de chaque spore

52

dissociée sur un milieu gélosé riche tel que milieu aux flocons d'avoine en boîte de Pétri;

b) au bout de 48 heures, on remet en suspension la totalité des spores de chaque boîte prise séparément et on ajuste le titre à $10^6$ spores/millilitre;

c) on prélève une ou plusieurs gouttes de suspension de souche obtenue après l'étape b) que l'on inocule dans l'un des milieux suivants :

i. un milieu gélosé pauvre, par exemple extrait de malt à 20 grammes par litre en boîte de Pétri que l'on cultive à 24°C;

ii. un milieu riche, par exemple aux flocons d'avoine à 30 grammes par litre, que l'on cultive à une température assez basse, par exemple de 15 à 17°C;

iii. un milieu de croissance du parasite Botrytis cinerea, par exemple essentiellement à base de concentré de jus de tomate, préalablement ensemencé du parasite, dans une boîte de Pétri, en vue de déterminer le pouvoir antagoniste vis-à-vis de ce parasite par contact et à distance avec culture à 24°C;

iiii. un essai sur milieu gélosé riche par exemple aux flocons d'avoine, pour déterminer les souches ayant la production la plus importante de spores par unité de surface du milieu gélosé;

d) on observe la croissance des souches à partir de 48 heures, toutes les 24 heures et on évalue selon des critères connus en soi la qualité de cette croissance;

e) on retient la ou les souches ayant obtenu la meilleure performance sur l'ensemble des essais et produisant la plus grande quantité de peptides, cette souche constitue la souche déposée auprès de l'Institut Pasteur sous le N° I223 et auprès de l'ATCC sous le N° 20672.

4. Procédé de culture de la souche Trichoderma harzianum selon la revendication 1 ou 2, ou obtenue selon le procédé selon la revendication 3, caractérisé en ce qu'il consiste à cultiver la souche de Trichoderma harzianum I223 ou ATCC N° 20672, sur un milieu liquide, composé de constituants nutritifs habituels en fermentation, stérilement, en couches fines sur un plateau et à la lumière pendant un temps de fermentation tel que les spores atteignent leur maturité; à la fin de la fermentation on sèche le produit total que l'on broie finement et que l'on tamise de manière à obtenir une poudre sèche et fine ayant de préférence un diamètre inférieur à 200 microns, contenant les spores vivantes, et pouvant se mettre en suspension dans l'eau et être pulvérisée.

5. Nouveaux peptides, caractérisés en ce qu'il s'agit de Trichorzianines isolées à partir des souches obtenues par clonage de champignons du type Trichoderma harzianum de préférence selon le procédé faisant l'objet de la revendication 3.

6. Nouveaux peptides selon la revendication 5, caractérisés en ce qu'il s'agit des Trichorzianines isolées à partir des spores obtenues avec le champignon Trichoderma harzianum I223 ou ATCC N° 20672.

7. Nouveaux peptides selon la revendication 5 ou 6, caractérisés en ce qu'il s'agit de la fraction

54

peptidique obtenue par extraction selon la procédé de la revendication 15 ou 16 du milieu de culture obtenu conformément au procédé de la revendication 4 et de préférence, constituée par la fraction III, IV, VI ou VII du chromatogramme de la figure 2.

8. Nouveaux peptides selon l'une quelconque des revendications 5 à 7, caractérisés en ce qu'il s'agit de la fraction majoritaire des Trichorzianines ayant un poids moléculaire égal à 1948 et donnant par hydrolyse acide dix-neuf acides aminés se décomposant comme suit :

huit Méthylalanines; deux Alanines; une Isoleucine; une Leucine; une Valine; une Proline; une Sérine; trois Glutamines; un Tryptophanol et les fractions minoritaires ayant au lieu du Tryptophanol un Phénylalaninol en position terminale.

9. Nouvelle souche de champignon Trichoderma harzianum Institut Pasteur N° I223 ou ATCC N° 20672 comme moyen de lutte biologique; par exemple comme produit phytosanitaire.

10. Produit obtenu, selon la revendication 4 comme moyen de lutte biologique, par exemple comme produit phytosanitaire.

11. Nouveaux peptides ou Trichorzianines selon l'une quelconque des revendications 5 à 8, et 24 à 27, de préférence obtenus à partir des spores de Trichoderma harzianum Institut Pasteur N° I223 ou ATCC N° 20672, caractérisés en ce qu'ils sont utilisés comme moyen de lutte biologique, en particulier sous forme de produit phytosanitaire.

55

12. Application de la nouvelle souche de Trichoderma harzianum Institut Pasteur N° I223 ou ATCC N° 20672 selon la revendication 1 ou 2, ou des Trichorzianines selon l'une des revendications 5 à 8 ou du produit obtenu par le procédé de culture selon la revendication 4, sous forme de produit phytosanitaire pour lutter contre le champignon parasite Botrytis cinerea.

13. Application de la souche de Trichoderma harzianum Institut Pasteur N° I223 ou ATCC N° 20672 selon la revendication 1 ou 2, ou des Trichorzianines selon l'une des revendications 5 à 8, ou du produit obtenu par le procédé de culture selon la revendication 4 sous forme de produit phytosanitaire pour lutter contre le champignon parasite Ceratocystis ulmi.

14. Application de la souche Trichoderma harzianum Institut Pasteur N° I223 ou ATCC N° 20672 selon la revendication 1 ou 2, ou des Trichorzianines selon l'une des revendications 5 à 8, ou du produit obtenu par le procédé de culture selon la revendication 4, pour lutter contre le champignon parasite Sclérotinia.

15. Application de la souche Trichoderma harzianum Institut Pasteur N° I223 ou ATCC N° 20672 selon la revendication 1 ou 2, ou des Trichorzianines selon l'une des revendications 5 à 8, ou du produit obtenu par le procédé de culture selon la revendication 4, sous forme de produit phytosanitaire pour lutter contre le champignon parasite Stereum purpureum ou Stereum hirsutum.

16. Application de la souche de Trichoderma harzianum Institut Pasteur N° I223 ou ATCC N° 20672 selon l'une quelconque des revendications 12 à 15, caractérisée en ce que ladite souche a été rendue résistante aux agents chimiques.

17. Procédé d'isolement des Trichorzianines par extraction des spores de Trichoderma harzianum I223 ou ATCC N° 20672 , caractérisé en ce qu'il comprend les étapes successives suivantes :

a)  introduire les spores de culture de Trichoderma harzianum Institut Pasteur N° I223 ou ATCC N° 20672 dans de l'acétone pour y dissoudre la partie soluble des spores et obtenir après évaporation de l'acétone un extrait brut;

b)  soumettre cet extrait brut à une étape d'extraction par l'éther pour en recueillir la fraction insoluble;

c)  extraire cette fraction insoluble dans l'éther au méthanol pour en recueillir la fraction soluble;

d)  chromatographier la fraction soluble dans le méthanol pour en recueillir la fraction de tête ou fraction lourde qui contient essentiellement les Trichorzianines.

18. Procédé selon la revendication 17, caractérisé en ce que l'on réalise l'étape d'extraction par chromatographie précitée, par chromatographie liquide haute performance en utilisant comme phase stationnaire de chromatographie une silice très fine et greffée par des hydrocarbures en C18 et en utilisant comme éluant un mélange éthanol-eau (65/35 v/v).

19.  Procédé d'isolement de pentyl-6 pyrone-2 par extraction des spores de Trichoderma harzianum I223 ou ATCC N° 20672, caractérisé en ce qu'il comprend l'étape de recueillir la fraction soluble dans l'éther obtenue à l'étape b) du procédé d'isolement selon la revendication 17; ainsi que l'étape d'isolement de la pentyl-6 pyrone-2 par tout procédé habituel, de préférence en utilisant une chromatographie sur colonne de silice avec comme éluant un mélange de pentane avec des quantités croissantes d'éther ou par chromatographie en phase vapeur.

20. Procédé d'isolement de pentyl-6 pyrone-2 par extraction des spores de Trichoderma harzianum I223 ou ATCC N° 20672, caractérisé en ce qu'il comprend les étapes successives suivantes :

a) introduire les spores de culture de Trichoderma harzianum Institut Pasteur N° I223 ou ATCC N° 20672 dans de l'hexane pour y dissoudre la partie soluble des spores et obtenir après évaporation de l'hexane un résidu huileux;

b) soumettre ce résidu huileux à une étape d'extraction par l'acétone ou le chlorure de méthylène pour en recueillir la fraction soluble;

c) soumettre directement la fraction soluble à une chromatographie en phase vapeur, ou évaporer l'acétone ou le chlorure de méthylène pour obtenir une huile orangée se gélifiant par refroidissement et qui contient la pentyl-6 pyrone-2 en mélange notamment avec de l'acide linoléique et soumettre ensuite ce mélange à une chromatographie sur colonne de gel de silice avec comme éluant un mélange de pentane avec des quantités croissantes d'éther pour en recueillir la fraction appropriée de pentyl-6 pyrone-2.

21. Utilisation de la pentyl-6 pyrone-2 comme moyen de lutte biologique, en particulier sous forme de produit phytosanitaire.

22. Application de la pentyl-6 pyrone-2 sous forme de produit phytosanitaire pour lutter contre le champignon parasite Botrytis cinerea.

23. Nouveau peptide caractérisé en ce qu'il s'agit de la Trichorzianine

Ac Aib Ala Ala Aib Aib Gln Aib Aib Aib Ser Leu Aib Pro Val Aib Ile Gln Gln Trpol

24. Nouveau peptide caractérisé en ce qu'il s'agit de la Trichorzianine

Ac Aib Ala Ala Aib Iva Cln Aib Aib Aib Ser Leu Aib Pro Val Aib Ile Gln Gln Phéol

25. Nouveau peptide, caractérisé en ce qu'il s'agit de la Trichorzianine

Ac Aib Ala Ala Aib Iva Gln Aib Aib Aib Ser Leu Aib Pro Leu Aib Ile Gln Gln Phéol

26. Nouveau peptide caractérisé en ce qu'il s'agit de la Trichorzianine

Ac Aib Ala Ala Aib Aib Gln Aib Aib Aib Ser Leu Aib Pro Val Aib Ile Gln Gln Phéol

0124388

1/13

Fig. 1

Val⁺  Leu⁺  Glu⁺  Pro⁺  Ala⁺  Aib⁺  Pheol⁺

Fig. 2

Fig. 3

2/13

0124388

H aromatiques du Phénylalaninol

δppm

8  7  6  5  4  3  2  1  0

RMN ¹H TCZ 11 A VI

Fig. 4

H aromatiques du Tryptophanol

δppm

8  7  6  5  4  3  2  1  0

RMN ¹H TCZ 11 A IV

C. aromatiques du phénylalaninol

Région des carbonyles

δppm

200    150    100    50    0

RMN $^{13}$C  TCZ 11 A VII

## Fig. 5

## Fig. 6

C. aromatiques du tryptophanol

Région des carbonyles

δppm

200    150    100    50    0

RMN $^{13}$C TCZ 11 A III

Région des C=O  RMN $^{13}$C  TCZ 11 A III

FIG. 7

4/13

0124388

Région des NH   RMN $^1$H  TCZ 11 A III

Fig. 9

CH₃OH dans le solvant

CH₃-CO-NH-

H aromatiques du tryptophanol

Région des H α

CH₂ β du tryptophanol

5ppm

8.0    7.0    4.0    3.0    2.0    1.0    0.0

RMN ¹H TCZ 11 A Ⅲ C

6/13

0124388

Fig. 10

RMN $^{13}$C  TCZ 11 A III C

0124388

7/13

Pro —Val —Aib —Ile —Gln —Gln —Phéol

REGION DES NH

H arom PHEOL

CONH₂ GLN

VAL AIB    GLN    GLN Pheol    ILE

PPM                8                    7

REGION DES Hα

VAL  PRO GLN ILE PHEOL    CH₂OH  CH₂N    Ψ CH₂         2CH₃ Aib    2CH₃Vel  2CH3 Ile
         CLN              PHEOL  PRO     PHEOL

δ PPM        4              3              2              1

FIG. 11   SPECTRE DE RMN ¹H (500 MHz DMSO·d₆) de l'oligopeptide Cop VII de TCZ 11A VII
          ATTRIBUTION DES NH ET Cα H

8/13

0124388

SPECTRE RMN $^1$H (500MHz, DMSO d6) de Cop VII Région des NH (ligne nférieure)

Fig. 12

SPECTRE DE RMN$^1$H (500 MHz, CD$_3$OD) DE LA TRICHORZIANINE TC Z 11A VI A

*FIG. 13*

0124388

SPECTRE DE RMN $^1$H (500 MHz CD$_3$OD) DE LA TRICHORZIANINE TCZ 11A VIA SPECTRE 2D DE CORRELATION PAR ECHO DE SPIN MISE EN EVIDENCE DES DEUX LEU (———(1) ET ..... (2)) ET DU PHENYLALANINOL (————)

ACIDE LINOLEIQUE→

←PENTYLPYRONE

**Fig. 15**

TEMPS
MN  50    40    30    20    10    0

Chromatogramme (phase vapeur) de l'extrait hexanique.
Chromatographe Carlo Erba type 21.50. Détection par ionisation de flamme.
Colonne capillaire 5/10, l = 20 m, OV 17 – gaz vecteur $N_2$ , débit
2,2 $cm^3$/min. 1° injecteur 280°C 1° détecteur 280°C. Programme température :
50°C/1 min. 50 ⟶ 120°C : 10°C/min – 120 ⟶ 280°C : 5°C/min.
Vitesse de déroulement du papier 5 mm/min.
Atténuation 8x10.

PENTYLPYRONE →

**Fig. 16**

TEMPS
MN    20    15    10    5    0

Chromatogramme (phase vapeur) de la pentylpyrone purifiée.
(conditions cf. Fig. 15)

Chromatogramme (phase vapeur) de l'extrait hexanique sur 1g de spores (conditions cf. Fig. 15)

Chromatographie (phase vapeur) de la fraction 7 (cf. Tabl.8) enrichie en pentylpyrone.

0124388

Office européen
des brevets

**RAPPORT DE RECHERCHE EUROPEENNE**

Numéro de la demande

EP 84 40 0545

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| P,X | JOURNAL OF MOLECULAR BIOLOGY, vol. 170, no. 3, 1983, Academic Press Inc. (London) Ltd., Londres (GB) B. BACHET et al.:"Crystallization and preliminary X-ray diffraction results of trichorzianine A 1, A peptide with nineteen residues from Trichoderma harzianum", pages 795-796<br>* Page 795, résumé; 1er alinéa; page 796, alinéa 2 *<br><br>--- | 1,5-8, 23 | C 12 N 1/14<br>C 12 P 21/02<br>C 12 P 17/06<br>C 07 C 103/52 //<br>(C 12 N 1/14<br>C 12 R 1/885)<br>(C 12 P 21/02<br>C 12 R 1/885)<br>(C 12 P 17/06<br>C 12 R 1/885) |
| P,X | BIOCHEMICAL AND BIOPHYSICAL RESEARCH COMMUNICATIONS, vol. 116, no. 1, 14 octobre 1983 D. DAVOUST et al.:"High field NMR study of antibiotic peptides: 1H assignment of trichorzianine A1 spectra by 2D experiments", pages 1-8<br>* Page 1, résumé, 1er alinéa; page 2:"Methods and materials" *<br><br>--- | 1,5-8, 23 | |
| A | EP-A-0 059 176 (MILJÖKONSULT ROLF HULTMAN AB)<br>* Revendications 1,5; page 6, lignes 22-31 *<br><br>---       -/- | 1,5,11 | **DOMAINES TECHNIQUES RECHERCHES (Int. Cl. 3)**<br><br>C 12 P<br>C 12 N<br>C 12 R<br>C 07 C |

Le présent rapport de recherche a été établi pour toutes les revendications

| Lieu de la recherche | Date d'achèvement de la recherche | Examinateur |
|---|---|---|
| LA HAYE | 15-08-1984 | DEKEIREL M.J. |

**Office européen des brevets**

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | CANADIAN JOURNAL OF MICROBIOLOGY, vol. 28, no. 7, 1982, National Research Council of Canada Y. ELAD et al.:"Degradation of plant pathogenic fungi by Trichoderma harzianum", pages 719-725 <br> * Page 719, résumé, "Introduction"; page 723, colonne de gauche, tableau 2 * | 1,5,11 | |
| A | JOURNAL OF AGRICULTURAL AND FOOD CHEMISTRY, vol. 20, no. 2, 1972, Washington D.C. (US) T. GREWELING et al.:"Characterization of the major aroma constituent of the fungus Trichoderma viride (Pers.)", pages 437-438 <br> * Page 437, résumé * | 19-21 | |
| A | CHEMICAL ABSTRACTS, vol. 97, no. 23, 6 décembre 1982, page 453, résumé no. 196817s, COLUMBUS, OHIO (US) & JP - A - 82 95 998 (SANKYO CO., LTD.) (15-06-1982) | | |
| A | JOURNAL OF THE SCIENCE OF FOOD AND AGRICULTURE, vol. 32, 1981, Society of Chemical Industry, Oxford (GB) P.J. MUINDI et al.:"Protein enrichment of Cassava Root Meal by Trichoderma harzianum for animal feed", pages 655-661 <br> --- -/- | | |

DOMAINES TECHNIQUES RECHERCHES (Int. Cl. ³)

Le présent rapport de recherche a été établi pour toutes les revendications

| Lieu de la recherche | Date d'achèvement de la recherche | Examinateur |
|---|---|---|
| LA HAYE | 15-08-1984 | DEKEIREL M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

Office européen
des brevets

**RAPPORT DE RECHERCHE EUROPEENNE**

| | **DOCUMENTS CONSIDERES COMME PERTINENTS** | | Page  3 |
|---|---|---|---|
| Catégorie | Citation du document avec indication, en cas de besoin, des parties pertinentes | Revendication concernée | CLASSEMENT DE LA DEMANDE (Int. Cl. ³) |
| A | FR-A-2 507 619  (NOVO INDUSTRI)<br><br>-----| | |

DOMAINES TECHNIQUES
RECHERCHES (Int. Cl. ³)

Le présent rapport de recherche a été établi pour toutes les revendications

| Lieu de la recherche | Date d'achèvement de la recherche | Examinateur |
|---|---|---|
| LA HAYE | 15-08-1984 | DEKEIREL M.J. |